Europäisches Patentamt

European Patent Office

Office européen des brevets

(19)

(11) Numéro de publication: **0 401 096**
**A1**

(12) **DEMANDE DE BREVET EUROPEEN**

(21) Numéro de dépôt: 90401398.4

(22) Date de dépôt: 23.05.90

(51) Int. Cl.5: **A61K 47/12, A61K 33/06**

(30) Priorité: 24.05.89 FR 8906819

(43) Date de publication de la demande:
05.12.90 Bulletin 90/49

(84) Etats contractants désignés:
**AT BE CH DE ES FR GB IT LI LU NL**

(71) Demandeur: **LABORATOIRES LUCIEN**
**3, rue des Ecoles**
**F-92700 Colombes(FR)**

Demandeur: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Berthon, Guy**
**La Bergerie**
**F-31320 Pechbusque(FR)**
Inventeur: **Gilbert, Huguette**
**12, Impasse du Clos Thiron**
**F-28630 Morancez(FR)**

(74) Mandataire: **Poidatz, Emmanuel**
**Cabinet M. SABATIER 83, Avenue Foch**
**F-75116 Paris(FR)**

(54) Agents et complexes de l'ion Mg2+ facilitant l'absorption du magnésium dans un organisme humain ou animal, et compositions pharmaceutiques ou diététiques utilisables pour l'administration de magnésium dans un organisme humain ou animal.

(57) Agents et complexes de l'ion magnésium Mg2+ facilitant l'absorption du magnésium dans un organisme humain ou animal, notamment agent-vecteur destiné à être associé à une solution monométallique de magnésium et comportant un ou plusieurs ligands de l'ion magnésium Mg2+ pour former au moins un complexe électriquement neutre, et administrable(s) dans l'organisme, la concentration en ligand(s) étant adaptée à la concentration choisie en ion Mg2+ pour assurer un pourcentage d'ions magnésium Mg2+ sous forme de complexe électriquement neutre au moins égal à 10 % en concentration molaire pour un pH compris entre 6,5 et 8.

FIG.22

## AGENTS ET COMPLEXES DE L'ION Mg2 + FACILITANT L'ABSORPTION DU MAGNESIUM DANS UN ORGANISME HUMAIN OU ANIMAL, ET COMPOSITIONS PHARMACEUTIQUES OU DIETETIQUES UTILISA-BLES POUR L'ADMINISTRATION DE MAGNESIUM DANS UN ORGANISME HUMAIN OU ANIMAL.

La présente invention concerne l'administration du magnésium à un organisme humain ou animal tant à titre de traitement, ou de prévention, de toutes les affections relevant de la magnésothérapie en général que dans le cadre de régimes diététiques humain et animal destinés à apporter du magnésium.

D'une façon générale, l'absorption dans un organisme humain d'une substance administrée par voie orale résulte de la faculté, pour cette substance, premièrement à traverser la membrane gastro-intestinale pour pénétrer dans le flux sanguin (absorption gastro-intestinale), deuxièmement à pénétrer dans les tissus à partir du plasma sanguin (absorption tissulaire).

Dans le cas particulier du magnésium, l'absorption gastro-intestinale est réalisée sans apport d'énergie par le double mécanisme de la diffusion passive et de la diffusion facilitée. La diffusion passive intervient suivant le gradient de concentration du magnésium qui doit cependant se présenter sous une forme non chargée électriquement pour assurer sa liposolubilité dans la membrane des entérocytes. La diffusion facilitée, au contraire de la diffusion passive, est un mécanisme saturable dans lequel le transport du magnésium intervient par l'intermédiaire d'un composé confiné dans la membrane traversée. Chez un sujet sain adulte, le partage diffusion passive diffusion facilitée est de l'ordre de 20 80 %. Toutefois, on a remarqué que chez les sujets dits malabsorbants, le pourcentage de diffusion facilitée chute de façon sensible, notamment chez les personnes agées, chez les sujets diabétiques ou insuffisants rénaux et chez les personnes suivant certains traitements thérapeutiques. Cette réduction de l'absorption gastro-intestinale se traduit par une augmentation du taux d'élimination du magnésium ingéré par excrétion fécale directe.

En ce qui concerne l'absorption tissulaire du magnésium, elle est le plus souvent médiocre, entrainant une élimination importante du magnésium par voie urinaire. Ici encore, l'absorption tissulaire est favorisée par l'augmentation de complexes de magnésium sous forme électriquement neutre, tandis qu'inversement l'accroîssement du magnésium (libre ou complexé) électriquement chargé doit entraîner une plus grande excrétion tissulaire.

Dans le cadre de la magnésothérapie ont déjà été proposées des compositions pharmaceutiques administrables par voie orale dans lesquelles l'ion Mg2 + est apparié à des anions tels que le sulfate et certains halogénures. A des concentrations thérapeutiques et dans des conditions de pH équivalentes au pH de la lumière gastro-intestinale. les ions Mg2 + se présentent sous leur forme unitaire chargée électriquement. Il en résulte, pour ces ions, une difficulté à diffuser de façon passive au travers des membranes des entérocytes et un taux important d'élimination du magnésium ingéré par excrétion fécale directe.

Ont également été proposées. dans le cadre de la magnésothérapie, des compositions pharmaceutiqués dans lesquelles le magnésium est uniquement administré sous la forme de sels stoechiométriques de magnésium tels par exemple les glutamate, aspartate, pidolate et lactate. Des études ont révélé que certains de ces sels étaient susceptibles dans les conditions du biofluide gastro-intestinal. de former avec l'ion Mg2 + des complexes électriquement neutres a priori plus facilement diffusibles au travers de la membrane intestinale grâce à leur absence de charge. Toutefois la présentation strictement stoechiométrique des sels de magnésium impose. par le jeu des équilibres chimiques, un pourcentage de complexes neutres trop faible pour avoir, en tant que complexe neutre, un effet spécifique significatif sur l'absorption du magnésium.

L'invention a essentiellement pour but d'améliorer l'absorption du magnésium dans un organisme humain ou animal par l'action de complexes neutres de l'ion Mg2 + au niveau de la diffusion ou rétention tissulaire en général. En particulier. l'invention concerne de nouveaux complexes de l'ion magnésium Mg2 +, de nouveaux agents facilitant l'absorption du magnésium et de nouvelles compositions pharmaceutiques ou diététiques utilisables pour l'administration de magnésium dans un organisme humain ou animal. Il est à noter que l'invention vise l'amélioration de l'absorption du magnésium dans un organisme humain ou animal. Dans le cas d'une administration orale, on recherche une amélioration de préférence aux deux niveaux, absorption gastro-intestinale et rétention tissulaire, mais également à un des deux niveaux, soit l'absorption gastro-intestinale, soit rétention tissulaire, dans la mesure où l'utilisation de l'agent-vecteur concerné n'entraîne pas une détérioration de l'absorption de magnésium à l'autre niveau. Dans le cas d'une administration parentérale, seule l'amélioration de la rétention tissulaire est visée. D'une façon générale est visée par l'invention la formation de complexes neutres de l'ion magnésium Mg2 + au pH de la lumière gastro-intestinale et ou au pH du plasma sanguin (pH = 7,4).

L'invention propose un agent-vecteur facilitant l'absorption d'une dose efficace de magnésium dans un organisme humain ou animal en tant que seul élément métallique actif, caractérisé en ce qu'il comporte un ou plusieurs ligands de l'ion magnésium Mg2+ pour former au moins un complexe électriquement neutre, et administrable(s) dans l'organisme, et en ce qu'il présente, pour être associé dans une solution monométallique à un ou plusieurs sels de magnésium, une concentration en ligand(s) adaptée à la concentration choisie en ion Mg2+ pour assurer un pourcentage d'ions magnésium Mg2+ sous forme de complexe électriquement neutre au moins égal à 10 % en concentration molaire pour un pH compris entre 6,5 et 8.

L'invention concerne également des compositions de pharmacie ou de diététique humaine et vétérinaire caractérisées en ce qu'elles contiennent du magnésium en tant que seul élément métallique actif et en ce qu'elles comportent au moins un agent-vecteur facilitant l'absorption du magnésium défini ci-dessus.

Avantageusement, l'agent-vecteur présent dans les compositions est associé à un composé ou sel ionique administrable dans l'organisme, facilement dissociable en solution aqueuse, susceptible de libérer l'ion Mg2+ en vue de sa complexation, notamment le chlorure de magnésium ou le carbonate de magnésium.

Selon une autre variante de l'invention, la composition comporte au moins 25 mg d'élément métallique magnésium, administrables en solution aqueuse et correspondant à une concentration minimale de 0,005 mol.dm-3 (mole par litre), associés à un agent-vecteur dont la concentration minimale assure un pourcentage d'ions magnésium sous forme de complexe électriquement neutre au moins égal à 10% en concentration molaire.

Il est à noter que, dans la présentation de l'invention donnée ci-dessus, l'agent-vecteur est utilisé pour l'absorption du magnésium en tant que seul élément métallique, permettant ainsi un meilleur contrôle des concentrations en ligand et en magnésium. Toutefois, l'invention n'est pas limitée à cette caractéristique et concerne également des agents-vecteurs du magnésium utilisables dans des solutions polymétalliques.

L'invention propose également un agent-vecteur facilitant l'absorption du magnésium dans un organisme humain ou animal et destiné à être administré soit en tant que seul apporteur de magnésium, soit en association avec un autre composé apporteur de magnésium, caractérisé en ce qu'il contient un ou plusieurs des composés suivants, ou de leurs sels d'addition administrables dans l'organisme, agissant en tant qu'apporteurs de ligand de l'ion Mg2+ : acide maléique, acide malonique et acide malique.

L'invention concerne également les compositions pharmaceutiques ou diététiques comportant au moins un agent-vecteur défini dans le paragraphe précédent éventuellement associé à un autre composé ou sel apporteur en magnésium, notamment le chlorure de magnésium ou le carbonate de magnésium, pour former avec l'ion Mg2+ au moins un complexe électiquement neutre. Avantageusement, ces compositions comportent au moins 25 mg d'élément métallique magnésium en solution aqueuse correspondant à une concentration minimum de 0,005 mol.dm-3 en association avec un agent-vecteur de concentration minimum prédéfinie.

Selon un mode de réalisation particulier d'une composition pharmaceutique ou diététique selon l'invention, l'agent- vecteur contient de l'acide (DL)-malique ou un de ses sels administrable dans l'organisme, la concentration en ligand (DL)-malate étant de préférence égale à 0,076 mol.dm-3 pour une concentration de magnésium égale à 0,04 mol.dm-3.

Selon un autre mode de réalisation particulier d'une composition de l'invention, l'agent-vecteur contient de l'acide (DL) - malique ou un de ses sels administrables dans l'organisme, le rapport des concentrations molaires ligand-malate/magnésium étant de préférence de l'ordre de 2.

Selon encore un autre mode de réalisation particulier de l'invention, est proposée une composition pharmaceutique ou diététique du type comportant l'ion citrate correspondant à une concentration de 0,005 mol.dm-3, pour former un complexe électriquement neutre d'ion Mg2+ selon une concentration minimale de 0,005 mol.dm-3 pour l'ion citrate seul.

Selon encore un autre mode de réalisation particulier de l'invention, est proposée une composition pharmaceutique ou diététique destinée à la magnésothérapie humaine ou vétérinaire et/ou aux supplémentations magnésiennes, la composition contenant en quantité efficace deux ingrédients actifs, distincts ou non, de l'acide citrique ou un de ses sels administrables dans l'organisme et un composé apporteur de l'ion Mg2+, en association avec un excipient ou un véhicule pharmaceutique acceptable.

Il est à noter que les compositions selon l'invention sont conditionnées sous la forme de préparations en vue d'une administration par voie orale ou parentérale.

L'invention concerne également à titre de produits nouveaux les complexes, notamment les complexes électriquement neutres, de l'ion Mg2+ de type MqLp et/ou de type MqLpHr où M représente l'ion Mg2+, H représente le proton et L représente un ligand choisi parmi le groupe suivant: maléate, malonate et malate.

Enfin l'invention concerne l'utilisation de ces nouveaux complexes à la magnésothérapie humaine ou

3

animale et ou aux supplémentations en magnésium dans le cadre de régimes diététiques humain et animal.

D'autres caractéristiques et avantages de l'invention seront mieux compris à la lecture de la description qui va suivre dans laquelle sont présentés, à titre d'exemples non limitatifs, quelques agents-vecteurs facilitant l'absorption du magnésium, les complexes correspondants et quelques compositions pharmaceutiques ou diététiques utilisables pour l'administration du magnésium dans un organisme humain ou animal. Ces exemples sont accompagnés de tableaux et de figures qui seront présentés en cours de l'exposé qui, pour des raisons de présentation n'ayant aucun caractère limitatif, a été divisé en quatre chapitres principaux "Recherche in vitro et simulation", "Résultats", "Acide malique", "Applications pharmaceutiques et diététiques".Par ailleurs, par convention dans le présent exposé, l'expression mol.dm-3 désigne une concentration en mole par litre et les expressions cm3 et ml désignent un volume en centimètre cube.


I - Recherche in vitro et simulation

D'une façon générale, toute substance chimique introduite dans un biofluide, par exemple le fluide gastro-intestinal, est soumise à des interactions de la part des autres substances qui le composent. Il en résulte que seule une fraction de cette substance demeure sous la forme initialement introduite. Le reste se répartit sous différentes formes complexes, dont les proportions respectives dépendent :
- de la force de ces interactions.
- des concentrations des divers réactifs en présence,
- du pH de la solution.

La capacité d'une substance à franchir les membranes cellulaires qui séparent le biofluide considéré des autres compartiments de l'organisme dépend en premier lieu de l'état électriquement neutre de ses formes prépondérantes. Ce dernier point, qui a déjà fait l'objet d'un développement particulier, ne sera pas repris ici. En ce qui concerne l'absorption du magnésium, l'invention comporte notamment l'utilisation après formation en quantité appréciable de complexes électriquement neutres de l'ion Mg2+ relativement stables au pH de la lumière gastro-intestinale. Il en résulte, dans un premier temps, un effet favorable au niveau de la composante diffusion passive de l'absorption gastro-intestinale. De plus, le vecteur facilitant l'absorption gastro-intestinale du magnésium diffuse également dans le plasma, sous sa forme protonée. pour atteindre une concentration suffisamment importante de sorte qu'il favorise la formation de complexes neutres de l'ion Mg2+ dans ce milieu (pH = 7,4) et par voie de conséquence, la diffusion tissulaire de ce métal.

La recherche des ligands susceptibles de constituer un agent-vecteur favorisant l'absorption d'une dose efficace de magnésium fait appel à la simulation informatique. En effet, si les techniques analytiques permettent de mesurer la concentration globale d'une substance donnée. elles ne permettent pas d'accéder aux valeurs individuelles des différentes formes auxquelles celle-ci donne naissance.

La constitution de modèles de simulation appropriés à chaque type de situation requiert l'identification des composants du biofluide, notamment des divers complexes de l'ion magnésium Mg2+ et la mesure de leurs concentrations globales, puis la détermination expérimentale des paramètres qui définissent la force des interactions, c'est-à-dire les constantes de formation des espèces complexes.

Pour ce faire, la méthode utilisée a consisté à :

1°' - déterminer in vitro les constantes d'équilibre des complexes magnésiens dans les conditions expérimentales adéquates, c'est-à-dire les plus proches des conditions physiologiques (par exemple à 37° C),

2°· - simuler la répartition des complexes magnésiens aux concentrations thérapeutiques dans le fluide gastro-intestinal entre pH 1 et 8, avec une attention particulière pour l'intervalle 6 à 7. Cette méthode a été présentée de façon générale dans un article de C. Blaquière et G. Berthon dans la revue Inorganica Chimica Acta. 135 (1987) 179-189 concernant l'étude de la formation de certains complexes de l'ion Mg2+ dans l'optique de son absorption gastro-intestinale. Sauf indications particulières. cet article précise les références des programmes informatiques utilisés pour la simulation.

3°' - simuler l'effet de l'absorption gastro-intestinale du ligand administré sur la répartition de la fraction non protéique de l'ion Mg2+ dans le plasma sanguin, sachant que l'augmentation de la part de magnésium complexé sous forme électriquement neutre doit améliorer son absorption tissulaire, tandis qu'inversement l'accroissement de la part de magnésium (libre ou complexé) électriquement chargé doit entraîner une plus grande excrétion urinaire.

La méthode de recherche de ligands a été utilisée pour divers vecteurs potentiels choisis parmi les anions de ligands essentiellement organiques susceptibles de former des complexes avec le magnésium. Les anions minéraux tels que le sulfate et les halogénures ont été d'emblée écartés car les complexes potentiels sont totalement dissociés aux concentrations thérapeutiques. Il est à noter que, pour la suite de la description, on suivra la convention d'écriture suivante de la formule générale d'un complexe MqLpHr avec M = métal, L = ligand, H = proton.

A titre d'exemple non limitatif, la méthode de détermination des constantes d'équilibre et de simulation de répartition des complexes de magnésium, est maintenant présentée en se référant au système magnésium-malonate.

I - Phase 1 : détermination des constantes de formation des complexes.

La méthode potentiométrique associée aux techniques informatiques a été utilisée pour cette étude. Les constantes de protonation du ligand, utilisées par la suite comme références, ont été calculées au moyen d'un programme informatique (MINIQUAD) à partir de données expérimentales provenant de titrages réalisés en solution aqueuse à 37°C et sous atmosphère d'azote.

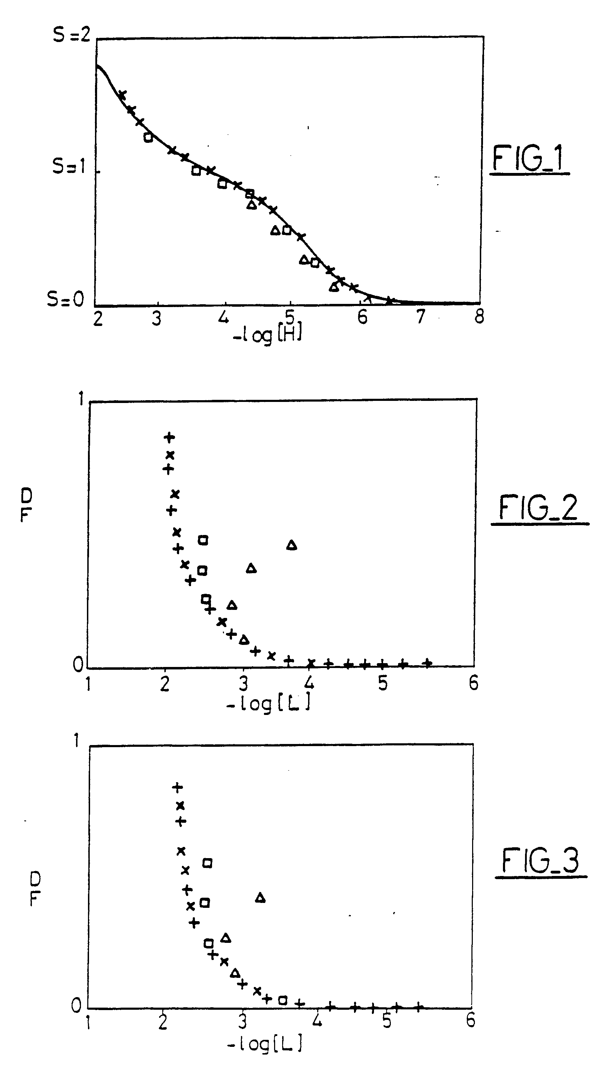

La complexation dans chaque système magnésium-ligand a ensuite été étudiée à partir de plusieurs titrages en solution aqueuse du ligand en présence de magnésium, pour différents rapports de concentrations métal-ligand correspondant à l'éventail le plus large possible de conditions thérapeutiques. Ces dosages se traduisent graphiquement par le tracé des courbes de protonation s = f ( -log[H] ), où s est le nombre moyen de protons liés au ligand. Ce type de courbes permet de visualiser la complexation. Par exemple, sur la figure 1, relative au système magnésium-malonate, on a représenté la courbe de protonation de référence du ligand par un trait plein et matérialisé les points expérimentaux des titrages du ligand en présence de magnésium par les symboles +, x, , □, ◁ dans l'ordre des rapports de concentrations métal ligand croissants. On remarque deux zones de pH différentes :

- l'une, de pH 2 à 3, pour laquelle les points se superposent à la courbe de protonation de référence, ce qui dénote une absence totale de complexation,

- l'autre, de pH 3 à 7, pour laquelle les points sont décalés vers la gauche par rapport à la protonation de référence, c'est-à-dire vers des pH plus acides. Il y a donc une libération d'ions H+ en solution due à la complexation des ions Mg2+ par le malonate.

Cette complexation est confirmée par le tracé de la courbe de formation expérimentale p = f ( -log [L] ) au moyen d'un autre programme (ZAPAPLOT) où p est le nombre moyen de ligands liés au métal (représenté par le degré de formation DF à la figure 2). L'allure de cette courbe donne une idée de la forme sous laquelle le ligand intervient dans les complexes formés et permet d'estimer la valeur de leurs constantes de formation.

A partir des données expérimentales des titrages, le programme MINIQUAD calcule les constantes par la méthode des moindres carrés. Parmi les différentes combinaisons de complexes possibles, seules sont retenues celles pour lesquelles les critères numériques (notamment la somme des carrés des résidus) sont convenables. Le meilleur ensemble est finalement choisi d'après le tracé des courbes de formation simulées au moyen d'un troisième programme informatique (PSEUDOPLOT) : c'est celui qui permet d'obtenir la meilleure coïncidence entre courbes expérimentale (figure 2) et simulée (figure 3).

Trois cas possibles a priori ont été effectivement rencontrés :

i) si aucune complexation n'est constatée dans les conditions expérimentales précisées plus haut, c'est à dire si le degré de complexation reste nul sur tout le domaine de concentrations en ligand examiné, aucune espèce complexe indépendante ne peut exister dans le fluide gastro-intestinal. Ceci ne signifie pas que les ligands envisagés ne puissent pas coordonner le magnésium à des concentrations beaucoup plus élevées, mais cette possibilité se rapportant à des conditions extra-physiologiques ne présente pas d'intérêt dans le cadre de l'invention.

ii) si l'ensemble de complexes sélectionné d'après les critères ci-dessus n'est constitué que d'espèces ionisées, il est impossible à ces dernières de se dissoudre dans la membrane phospholipidique ; les ligands correspondants ne peuvent donc être utilisés comme vecteurs pour l'absorption gastro-intestinale du magnésium.

iii) si l'ensemble retenu contient une ou plusieurs espèces neutres, les ligands correspondants peuvent être a priori des vecteurs efficaces pour l'absorption du magnésium par les membranes séparant le biofluide d'un biofluide voisin. Leur efficacité dépendra toutefois de la proportion de ces espèces neutres dans les conditions thérapeutiques. L'étude de ces systèmes est étendue à l'analyse de la complexation du calcium par le même ligand, afin de tester la possibilité d'une éventuelle compétition antagoniste.

En ce qui concerne le malonate, les constantes (log β) de protonation des ligands et de formation des complexes correspondantes sont données dans le Tableau 1 suivant :

| Système | Complexe MqLpHr | log $\beta$ | + - | Charge |
|---------|----------------|-------------|------|--------|
| proton-malonate | LH | 5,235 | 0,002 | - |
| proton-malonate | LH2 | 7,815 | 0,003 | 0 |
| magnésium-malonate | ML | 2,064 | 0,007 | 0 |
| magnésium-malonate | ML3 | 5,266 | 0,043 | 4- |
| magnésium-malonate | MLH | 5,524 | 0,118 | + |
| magnésium-malonate | MLH-1 | 6,326 | 0,099 | - |
| calcium-malonate | ML | 1,511 | 0,009 | 0 |

I - Phase 2 : Simulation dans le fluide gastro-intestinal.

Une fois le meilleur ensemble de complexes établi pour chaque système potentiellement actif, il reste à déterminer les conditions de concentrations susceptibles d'entraîner la formation d'un pourcentage optimal d'espèce(s) neutre(s) dans le fluide gastro-intestinal. Dans le cas de systèmes simples. ne comportant par exemple qu'un complexe monomère de type ML et son homologue protoné MLH. la connaissance des constantes "conditionnelles" de formation de ces espèces permettrait d'en situer les pourcentages respectifs. Pour des systèmes faisant intervenir plusieurs complexes tels que ceux envisagés ici. le pourcentage d'espèce neutre dépend non seulement des concentrations en ions $Mg2+$ et en ligand, du rapport métal ligand, mais aussi du pH. On a alors recours à la simulation par ordinateur. et c'est grâce aux programmes informatiques (COMICS, COMPLOT ou et TRIPLOT) spécialement élaborés à cet effet que l'on peut déterminer les conditions optimales d'administration : il s'agit de calculer la répartition théorique des espèces formées dans les conditions de concentration de la lumière gastro-intestinale en fonction du pH.

L'appréciation dans la pratique de la distribution réelle d'un médicament administré par voie orale pose un certain nombre de questions discutées ci-dessous.

L'administration de complexes métalliques peut se faire :

- soit dans un estomac vide, pratique souhaitable ; cette solution offre un avantage notable : les sécrétions gastro-intestinales spécifiques au processus de digestion peuvent être négligées et la répartition de l'ion métallique en ses différents complexes avec le ligand choisi peut être simulée directement.

- soit dans un estomac plein. au moment des repas : c'est ce qui est habituellement recommandé pour les médicaments actuels contenant du magnésium. Dans ce cas. la simulation de la répartition réelle du métal pose un problème beaucoup plus ardu. spécifique à chaque situation particulière : il y a compétition entre les ligands libérés au cours de la digestion et ces mêmes vecteurs vis-à-vis de l'ion $Mg2+$.

Cette deuxième possibilité ne peut donc. par définition. être développée sur le plan général. Néanmoins, pour prendre en compte les variations dans différents volumes de fluide gastro-intestinal, deux concentrations de magnésium ont été considérées pour les simulations : 0,005 et 0,05 mol.dm-3, avec une concentration de ligand associé telle que le rapport métal ligand varie de 1 1 à 1 10. Par exemple. dans le cas du malonate (désigné sous le symbole MLO). les simulations des figures 4a b c d mettent en évidence un comportement du système magnésium-malonate très variable en fonction des concentrations et des rapports de concentrations métal ligand.

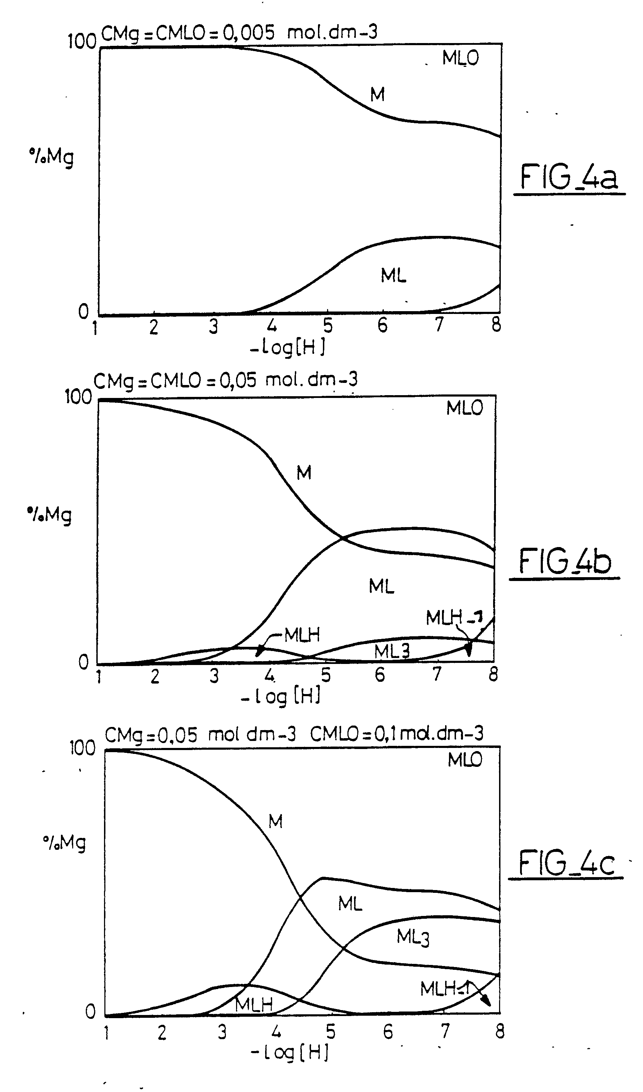

L'examen des quatre courbes de simulation présentées aux figures 4a b c d montre que l'espèce neutre est favorisée pour CMg = 0,05 mol.dm-3 et CMLO = 0,1 mol.dm-3. Dans ce cas (figure 4c), ML existe de pH 3 à 8 avec un maximum de 51% à pH 5 et se maintient de pH 5 à 8 aux environs de 45%.

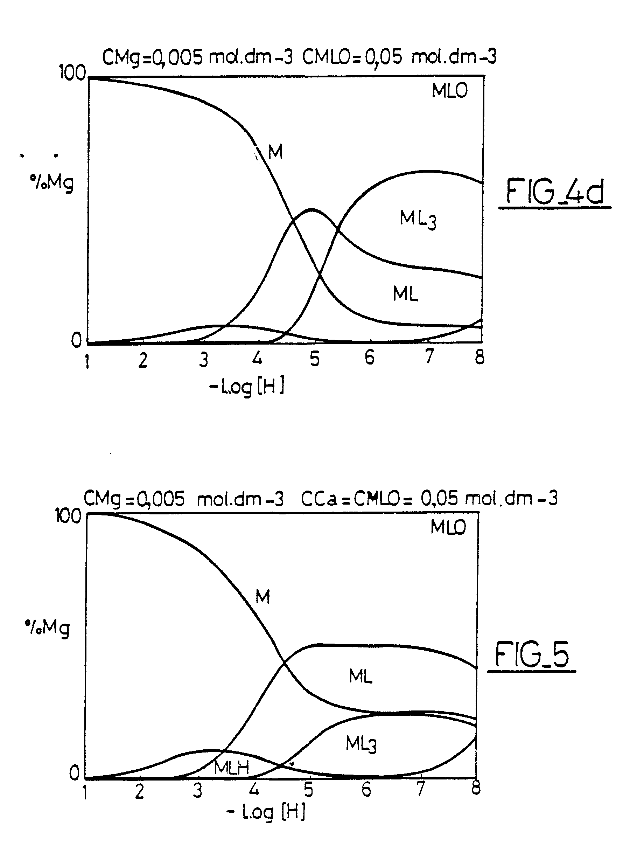

Par la suite, les interactions potentielles du calcium sur la mobilisation du magnésium en espèces neutres ont été analysées en simulant tout d'abord des concentrations de calcium équivalentes à celle du magnésium puis jusqu'à 10 fois supérieure. Les résultats ont montré que le calcium a une influence positive sur le pourcentage d'espèces magnésiennes neutres incorporant le ligand malonate (voir la figure 5 à rapprocher de la figure 4d).

I - Phase 3 : Simulation dans le plasma sanguin.

L'anion malonate ayant été reconnu ci-dessus comme un vecteur possible pour l'ion $Mg2+$ du fluide gastro-intestinal vers le plasma sanguin, la simulation de l'influence d'un accroissement de la concentration de cet anion sur la répartition du magnésium plasmatique non lié aux protéines a été effectuée.

Pour ce faire, les constantes de formation communiquées dans le Tableau 1 ont en premier lieu été incorporées dans une banque de données relative au plasma sanguin. Les limites de variation de la concentration de malonate dans le plasma ont ensuite été fixées entre 10-5 et 10-2 mol.dm-3, ce qui correspond respectivement à 5,203 mg et 5,203 g de malonate dissous dans 5 dm3 (5 litres) de plasma sanguin. Ainsi qu'on le verra plus loin, ces doses encadrent très largement les quantités thérapeutiques administrables. La concentration d'ions Mg2 + libres dans le plasma, égale à 0,520.10-3 mol.dm-3, ainsi que le pH, égal à 7,4, ont été maintenus constants au cours des simulations réalisées au moyen du programme ECCLES (P.M. May, T.W. Linder and D.R. Williams, J. Chem. Soc., Dalton Trans., 588-595 (1977)).

Les résultats sont consignés dans le Tableau 2, où l'on constate que la fraction de magnésium plasmatique complexé sous forme neutre augmente dans des proportions notables sous l'effet du malonate. Elle est approximativement doublée vers 1.10-3 mol.dm-3 (0,001 mole par litre), ce qui correspondrait à une dose orale totalement absorbée de 0,520 g de malonate.

TABLEAU 2

| CMLO (mol.dm-3) | % Mg | % NMg | Accroissement de NMg (en %) |
|---|---|---|---|
| 1,00.10-5 | 75,3 | 7.8 | 0.0 |
| 3,16.10-5 | 75,1 | 8.0 | 2.6 |
| 1,00.10-4 | 74,7 | 8.6 | 10.2 |
| 3,16.10-4 | 73,3 | 10.1 | 29.5 |
| 1,00.10-3 | 69,3 | 14.4 | 84.6 |
| 3,16.10-3 | 59,0 | 25.5 | 226.9 |
| 1,00.10-2 | 38,7 | 43.6 | 459.0 |

où % Mg représente le pourcentage de la fraction de Mg sous forme de métal libre par rapport à la fraction ultra filtrable disponible (non lié aux protéines) de Mg dans le plasma, et
où % NMg représente le pourcentage de la fraction de la totalité de complexes neutres de l'ion Mg2 + (NMg) par rapport à la fraction ultra filtrable disponible.

II - Résultats :

Au terme de la première phase de cette recherche, les résultats présentés ci-après ont permis de retenir à titre d'agent-vecteur favorisant l'absorption, notamment gastro-intestinale, du magnésium les ligands suivants comme formant avec le magnésium des complexes relativement stables avec au moins une espèce neutre : ce sont, outre l'anion malonate (MLO), les anions glutamate (GLU), aspartate (ASP), citrate (CTA), phosphate (PO4), $\alpha$ et $\beta$-glycérophosphate (AGC et BGC), phosphate de pyridoxal (PDP), tartrate (TRA), maléate (MLE), éthylmalonate (EMA) et malate (MLA). De plus, des complexes très stables, dont des espèces neutres, ont été mis en évidence avec les quatre ligands suivants également susceptibles d'être utilisés comme agent-vecteur: acide iminodiacétique (IDA), tropolone (TPL) (2-hydroxy-2,4,6-cycloheptatrienone), tiron (TIR) (acide 4,5-dihydroxy-1.3-benzène-disulfonique) et acide kojique (KJA) (5-hydroxy-2-(hydroxy-méthyl)-4H-pyran-4-one).

Il est à noter que, parmi toutes les préparations utilisant ces composés, celles destinées à des applications pharmaceutiques ou diététiques seront présentées sous une forme pratiquement ingérable et à des concentrations non-toxiques selon les critères en vigueur dans la profession, après les vérifications d'usage bien connues de l'homme de l'art.

Les Tableaux 3 et 4 présentés en pages suivantes, donnent les valeurs des constantes de protonation des ligands et des constantes d'équilibre de formation des complexes de magnésium de type MqLpHr concernant les ligands sélectionnés calculées à partir des résultats expérimentaux obtenus par titrage.

D'une façon générale, les complexes sont identifiés par les valeurs de leurs indices de coordination p, q et r.

## TABLEAU 3

| Système | p | q | r | log β | ± |
|---|---|---|---|---|---|
| Proton-glutamate | 1 | 0 | 1 | 9,175 | |
| | 1 | 0 | 2 | 13,256 | |
| | 1 | 0 | 3 | 15,440 | |
| Proton-aspartate | 1 | 0 | 1 | 9,321 | 0,021 |
| | 1 | 0 | 2 | 12,935 | 0,005 |
| | 1 | 0 | 3 | 14,910 | 0,006 |
| Proton-citrate | 1 | 0 | 1 | 5,513 | 0,002 |
| | 1 | 0 | 2 | 9,758 | 0,003 |
| | 1 | 0 | 3 | 12,581 | 0,004 |
| Proton-tartrate | 1 | 0 | 1 | 3,855 | 0,002 |
| | 1 | 0 | 2 | 6,584 | 0,002 |

TABLEAU 3

## TABLEAU 3 (suite)

| Système | p | q | r | log β | ± |
|---|---|---|---|---|---|
| Proton-phosphate | 1 | 0 | 1 | 6,627 | 0,002 |
| | 1 | 0 | 2 | 8,575 | 0,004 |
| Proton-pyridoxal phosphate | 1 | 0 | 1 | 7,948 | 0,002 |
| | 1 | 0 | 2 | 13,904 | 0,003 |
| | 1 | 0 | 3 | 17,320 | 0,005 |
| Proton-α-glycérophosphate | 1 | 0 | 1 | 6,116 | 0,001 |
| | 1 | 0 | 2 | 7,311 | 0,011 |
| Proton-β-glycérophosphate | 1 | 0 | 1 | 6,143 | 0,002 |
| | 1 | 0 | 2 | 7,460 | 0,013 |
| Proton-maléate | 1 | 0 | 1 | 5,803 | 0,002 |
| | 1 | 0 | 2 | 7,525 | 0,004 |
| Proton-malonate | 1 | 0 | 1 | 5,235 | 0,002 |
| | 1 | 0 | 2 | 7,815 | 0,003 |
| Proton-éthyl-malonate | 1 | 0 | 1 | 5,409 | 0,001 |
| | 1 | 0 | 2 | 8,180 | 0,001 |
| Proton-malate | 1 | 0 | 1 | 4,608 | 0,001 |
| | 1 | 0 | 2 | 7,782 | 0,011 |
| Proton-iminodiacétate | 1 | 0 | 1 | 9,060 | 0,002 |
| | 1 | 0 | 2 | 11,611 | 0,006 |
| | 1 | 0 | 3 | 13,298 | 0,013 |
| Proton-kojate | 1 | 0 | 1 | 7,596 | 0,001 |
| Proton-tropolonate | 1 | 0 | 1 | 6,617 | 0,001 |
| Proton-tironate | 1 | 0 | 1 | 11,552 | 0,005 |
| | 1 | 0 | 2 | 18,914 | 0,005 |
| Proton-D-malate | 1 | 0 | 1 | 4,620 | 0,001 |
| | 1 | 0 | 2 | 7,819 | 0,002 |
| Proton-DL-malate | 1 | 0 | 1 | 4,615 | 0,001 |
| | 1 | 0 | 2 | 7,829 | 0,001 |
| Proton-DL-malate (usine) | 1 | 0 | 1 | 4,617 | 0,001 |
| | 1 | 0 | 2 | 7,818 | 0,001 |
| Proton-αβ-glycéro phosphate | 1 | 0 | 1 | 6,170 | 0,002 |
| | 1 | 0 | 2 | 7,507 | 0,013 |

9

## TABLEAU 4

| Système | p | q | r | logβ | ± |
|---|---|---|---|---|---|
| Magnésium-glutamate | 1 | 1 | 0 | 2,196 | 0,021 |
| | 1 | 1 | 1 | 11,081 | 0,018 |
| | 1 | 1 | 2 | 14,876 | 0,021 |
| | 2 | 1 | -1 | -6,125 | 0,023 |
| Magnésium-aspartate | 1 | 1 | 0 | 2,040 | 0,029 |
| | 1 | 1 | 1 | 10,501 | 0,030 |
| | 1 | 1 | 2 | 14,074 | 0,042 |
| | 1 | 1 | -1 | -8,666 | 0,023 |
| | 1 | 2 | 0 | 4,426 | 0,049 |
| Magnésium-citrate | 1 | 1 | 0 | 3,333 | 0,009 |
| | 1 | 1 | 1 | 7,483 | 0,024 |
| | 2 | 2 | -2 | -12,638 | 0,033 |
| | 1 | 1 | -2 | -18,468 | 0,015 |
| | 2 | 1 | 0 | 5,126 | 0,041 |
| | 2 | 1 | 1 | 10,411 | 0,069 |
| | 1 | 1 | 2 | 11,008 | 0,063 |
| Magnésium-tartrate | 1 | 1 | 0 | 1,202 | 0,013 |
| | 2 | 1 | 0 | 3,057 | 0,032 |
| | 1 | 1 | 1 | 4,120 | 0,111 |
| | 2 | 1 | -1 | -3,764 | 0,156 |
| Magnésium-phosphate | 1 | 1 | 0 | 2,399 | 0,071 |
| | 1 | 1 | 1 | 8,662 | 0,096 |
| Magnésium-pyridoxal phosphate | 1 | 1 | 0 | 2,050 | 0,014 |
| | 1 | 1 | 1 | 9,405 | 0,023 |
| | 2 | 1 | 1 | 11,628 | 0,106 |
| Magnésium-β-glycéro phosphate | 1 | 1 | 0 | 2,450 | 0,023 |
| | 1 | 1 | 1 | 8,338 | 0,026 |
| | 2 | 1 | 0 | 4,255 | 0,024 |
| | 2 | 1 | 1 | 10,114 | 0,032 |
| | 1 | 1 | -1 | -6,425 | 0,033 |
| Magnésium-α-glycéro phosphate | 1 | 1 | 0 | 1,896 | 0,023 |
| | 1 | 1 | 1 | 7,711 | 0,032 |
| | 2 | 1 | 0 | 3,609 | 0,025 |
| | 2 | 1 | 1 | 9,583 | 0,030 |
| | 1 | 1 | -1 | -8,083 | 0,291 |
| Magnésium-maléate | 1 | 1 | 0 | 1,803 | 0,015 |
| | 1 | 1 | 1 | 7,117 | 0,024 |
| | 2 | 1 | 0 | 2,678 | 0,194 |
| Magnésium-malonate | 1 | 1 | 0 | 2,064 | 0,007 |
| | 3 | 1 | 0 | 5,266 | 0,043 |
| | 1 | 1 | 1 | 5,524 | 0,118 |
| | 1 | 1 | -1 | -6,326 | 0,099 |

## TABLEAU 4 (suite)

| Système | p | q | r | logβ | ± |
|---|---|---|---|---|---|
| Magnésium-éthylmalonate | 1 | 1 | 0 | 1,601 | 0,018 |
| | 2 | 1 | 0 | 2,935 | 0,076 |
| | 1 | 1 | 1 | 5,904 | 0,078 |
| Magnésium-iminodiacétate | 1 | 1 | 0 | 2,876 | 0,007 |
| | 1 | 1 | 1 | 9,540 | 0,097 |
| | 2 | 1 | -1 | -6,146 | 0,041 |
| | 3 | 1 | 0 | 6,229 | 0,050 |
| Magnésium-kojate | 1 | 1 | 0 | 2,883 | 0,002 |
| | 2 | 1 | 0 | 4,997 | 0,003 |
| | 1 | 1 | -1 | -7,993 | 0,004 |
| Magnésium-tropolonate | 1 | 1 | 0 | 3,786 | 0,002 |
| | 2 | 1 | 0 | 6,805 | 0,003 |
| | 3 | 1 | 0 | 8,725 | 0,012 |
| Magnésium-tironate | 1 | 1 | 0 | 6,025 | 0,002 |
| | 2 | 1 | 0 | 9,653 | 0,008 |
| | 1 | 1 | 1 | 13,576 | 0,006 |
| | 2 | 1 | -1 | -1,688 | 0,008 |
| Magnésium-malate | 1 | 1 | 0 | 1,707 | 0,011 |
| | 1 | 1 | 1 | 5,579 | 0,025 |
| | 2 | 1 | 0 | 2,878 | 0,081 |
| Magnésium-D-malate | 1 | 1 | 0 | 1,707 | 0,007 |
| | 1 | 1 | 1 | 5,452 | 0,021 |
| | 2 | 1 | 0 | 2,864 | 0,056 |
| Magnésium-DL-malate | 1 | 1 | 0 | 1,712 | 0,007 |
| | 1 | 1 | 1 | 5,563 | 0,017 |
| | 2 | 1 | 0 | 2,837 | 0,059 |
| Magnésium-DL-malate (usine) | 1 | 1 | 0 | 1,738 | 0,005 |
| | 1 | 1 | 1 | 5,589 | 0,012 |
| | 2 | 1 | 0 | 2,862 | 0,044 |
| Magnésium-αβ-glycéro phosphate | 1 | 1 | 0 | 2,294 | 0,024 |
| | 1 | 1 | 1 | 8,066 | 0,034 |
| | 2 | 1 | 0 | 3,674 | 0,049 |
| | 2 | 1 | 1 | 9,825 | 0,052 |
| | 1 | 1 | -1 | -6,863 | 0,027 |

Il est à noter que les Tableaux 3 et 4 incorporent des résultats concernant diverses variétés des ligands malate qui se sont avérés particulièrement intéressants dans le cadre de la présente invention et qui seront discutés en détail ultérieurement.

Les résultats concernant la simulation de la répartition des complexes en fonction du pH sont détaillés ci-après en se référant aux figures correspondantes 6 à 21. Ces simulations effectuées dans les conditions de concentrations du fluide gastro-intestinal en fonction du pH permettent de mettre en évidence non seulement le pourcentage d'espèce neutre mais aussi la zone d'existence de cette espèce. Parmi tous les ligands étudiés formant des complexes neutres avec le magnésium, ont été écartés les ligands dont le pourcentage d'espèce neutre était trop faible ou ceux dont l'existence de l'espèce neutre se situait en dehors d'un intervalle d'utilisation prévue de pH compris entre 6/7,5.

Sauf indications contraires, les simulations présentées ci-après ont été effectuées pour CMg = 0,005 mol.dm-3 et CL = 0,05 mol.dm-3 :

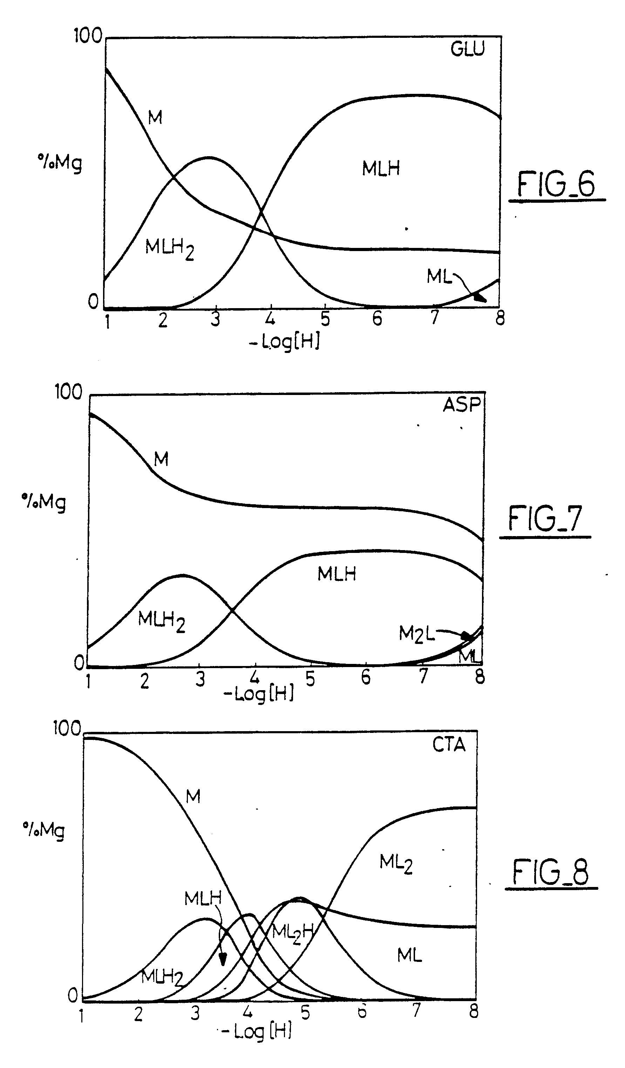

- Pour le glutamate (GLU) et l'aspartate (ASP), l'espèce neutre ML n'existe qu'à partir de pH 7 et en faible pourcentage (8% à pH 8) (figures 6 et 7).

- Avec le citrate (CTA), l'espèce neutre MLH existe de pH 2 à 6 avec un maximum de seulement 32% à pH 4 (figure 8).

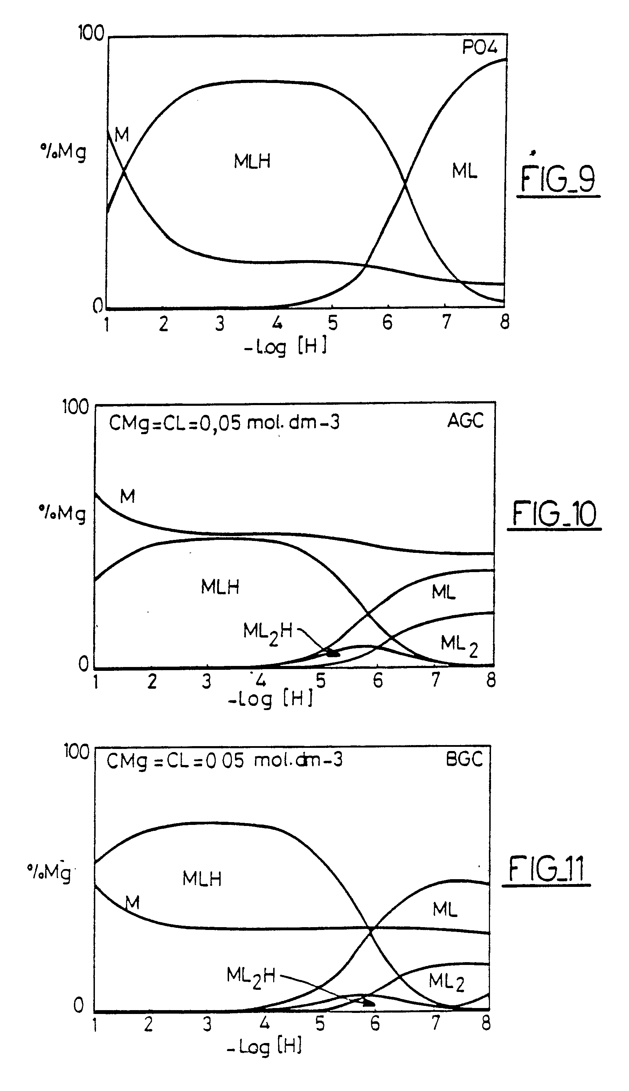

- L'ion phosphate (PO4) forme une espèce neutre dès pH 4, atteignant 91% à pH 8 (figure 9). De plus, la simulation de la répartition de l'espèce magnésienne neutre en présence de calcium est pratiquement identique à celle obtenue sans calcium, indiquant que le calcium n'a aucune influence sur la mobilisation du magnésium par le phosphate.

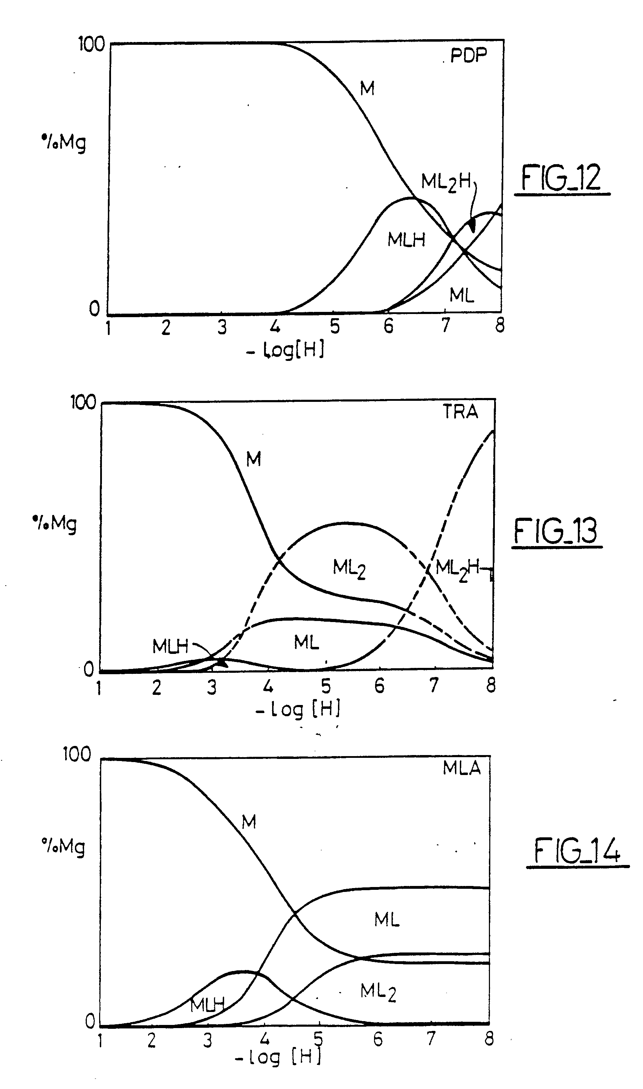

- Le glycérophosphate (étudié avec CMg = CL = 0,05 mol.dm-3) forme également une espèce neutre à partir de pH 4, atteignant à pH 8, 35% pour la forme $\alpha$ (AGC) (figure 10), et 47% pour la forme $\beta$ (BGC) (figure 11). L'influence du calcium apparaît également négligeable pour des concentrations en magnésium et calcium équivalentes.

Les sels $\alpha$ et $\beta$ -glycérophosphate sont deux produits de structures différentes ; l'étude de ces deux systèmes a conduit à sélectionner la forme $\beta$ au détriment de la forme $\alpha$.

Le produit $\alpha\beta$ -glycérophosphate (ABG) est un mélange équimoléculaire de ces deux formes. Toutefois, la pratique a montré que l'équimolécularité n'est généralement pas respectée dans les produits couramment disponibles. Sous cette réserve, les résultats de simulation présentés dérivent tous de l'étude in vitro du même composé -glycérophosphate. Les valeurs des constantes de protonation de ce composé se situent entre celles des produits purs $\alpha$ et $\beta$ (Tableau 3). De même, la stabilité des complexes du magnésium avec le composé $\alpha\beta$, notamment celle de ML, est intermédiaire entre les stabilités des complexes formés par les produits purs $\alpha$ et $\beta$ (Tableau 4).

En ce qui concerne la répartition de l'espèce neutre en fonction du pH et de la concentration en ligand dans les conditions du fluide gastro-intestinal, le pourcentage maximum, 56 % est obtenu à pH 7,5, lorsque, pour une concentration en magnésium de 0,04 mol.dm-3, la concentration en ligand est de 0,048 mol.dm-3. En présence d'une concentration en calcium égale à celle de magnésium (0,04 mol.dm-3), on obtient le même pourcentage pour une concentration en ligand de 0,104 mol.dm-3.

- Avec le tartrate (TRA), l'espèce magnésienne neutre existe dans une zone très étendue (pH 3 à 7) mais avec un pourcentage très faible (19%). C'est l'espèce chargée ML2 qui est majoritaire (figure 13).

- Le phosphate de pyridoxal (PDP) forme avec le magnésium une espèce neutre MLH de pH 5 à 8 avec un maximum de 43% à pH 6,2. Il faut toutefois signaler que les espèces chargées ML et ML2H cessent d'être négligeables dès pH 7 (figure 12).

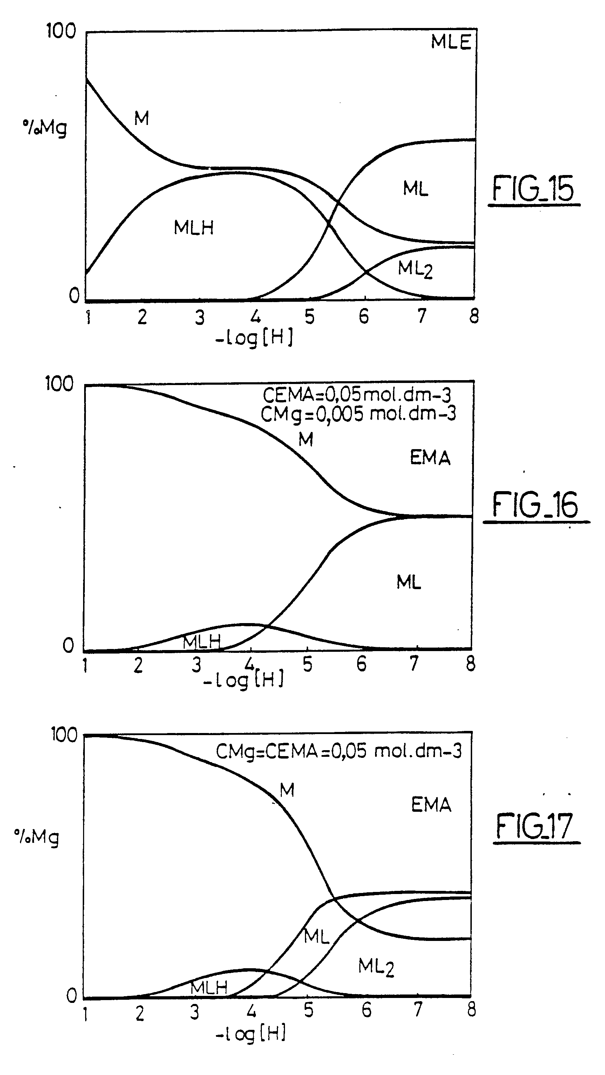

- Avec le malate (MLA) et le maléate (MLE), on obtient le même type de simulation : existence de l'espèce neutre ML avec un pourcentage de 51% de pH 5 à 8 pour le malate (figure 14) et de 60% de pH 6 à 8 pour le maléate (figure 15). Ces deux systèmes ne sont pas perturbés par la présence de calcium : les simulations de la répartition des espèces magnésiennes neutres en présence de calcium sont pratiquement inchangées pour des concentrations en calcium égales à celles de magnésium.

- Pour le malonate (MLO), on se reportera aux résultats présentés ci-avant.

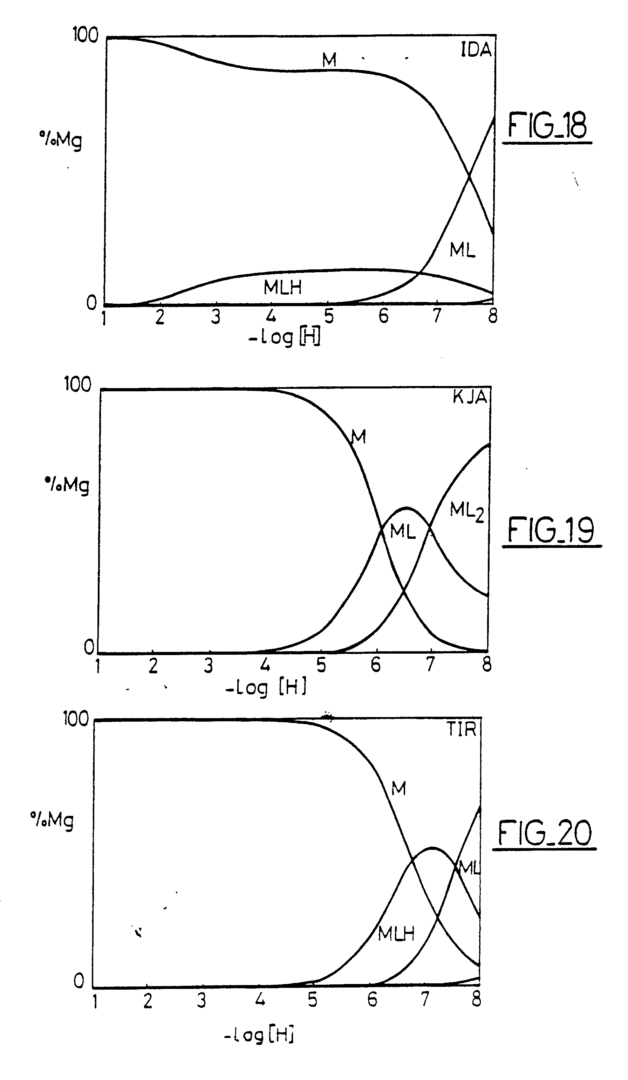

- En ce qui concerne l'éthylmalonate (EMA), la simulation permet aussi de déterminer les meilleures conditions de concentration pour optimiser le pourcentage d'espèce neutre. Alors que pour CMg = 0,005 et CEMA = 0,05 mol.dm-3, ML atteint 40% de pH 5 à 8 et ML2 avec 37% de pH 6 à 8 (figure 17), pour CMg = CEMA = 0,05 mol.dm-3, seule l'espèce ML est présente de pH 4 à 8 avec 50% de 6 à 8 (figure 16).

- L'ion iminodiacétate (IDA) forme avec le magnésium une espèce neutre ML existant uniquement de pH 6 à 8 avec un pourcentage maximum de 68% à pH 8 (figure 18).

- Avec l'acide kojique (KJA), l'espèce neutre ML2 est favorisée pour le rapport de concentration 1 10 avec CMg =0,005 mol.dm-3 : elle existe de pH 6 à 8 avec un maximum de 79% à pH 8 (figure 19).

- Le tiron (TIR) forme l'espèce neutre ML de pH 6 à 8 avec 67% à pH 8, mais l'espèce chargée MLH est présente de pH 8 à 8 avec un maximum à pH 7 (51%) (figure 20).

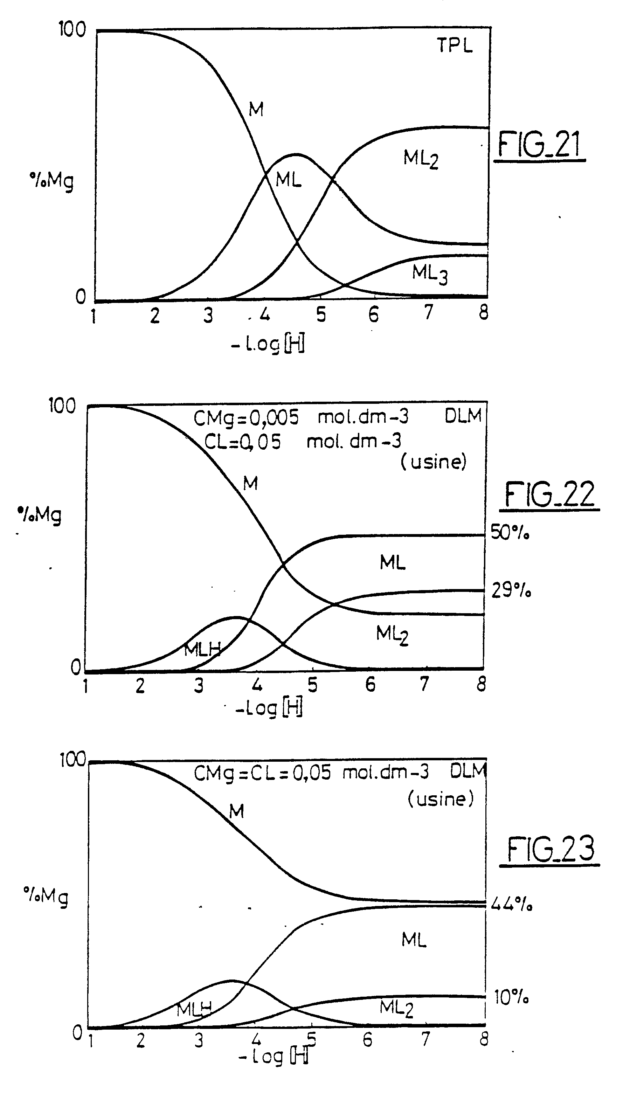

- En ce qui concerne le tropolone (TPL), l'espèce neutre ML2 est majoritaire pour CMg = 0,05 et CTPL = 0,1 mol.dm-3 : 63% de pH 6 à 8 (figure 21).

L'influence du calcium sur la répartition de l'espèce neutre magnésienne relative à ces quatre derniers ligands est négligeable.

L'étape de simulation Phase 2 est très intéressante puisqu'elle permet non seulement de détecter les meilleurs ligands susceptibles de vectoriser le magnésium, mais aussi de déterminer des conditions favorables d'administration orale en solution diluée de ce métal. Le Tableau 5 donne la répartition ou fraction maximum calculée de l'espèce neutre en fonction du pH et de la concentration en ligand pour une concentration de magnésium correspondant à 100 mg dans 100 cm3 (CMg = 0,04 mol.dm-3) et les valeurs optimales correspondantes du pH et de la concentration en ligand CL donnée en mol.dm-3. Dans ce tableau les abréviations DMA et DLM désignent respectivement l'acide (D)-malique et l'acide racémique (DL)-malique.

Tableau 5

| Ligand | Fraction maximum | pH | Concentration en ligand |
|--------|------------------|-----|--------------------------|
| GLU | 0,11 | 8 | 0,16 |
| ASP | 0,11 | 8 | 0,16 |
| CTA | 0,35 | 3,5 | 0,16 |
| TRA | 0,19 | 4,5 | 0,076 |
| PO4 | 0,95 | 8 | 0,16 |
| PDP | 0,42 | 8 | 0,076 |
| AGC | 0,37 | 8 | 0,048 |
| BGC | 0,49 | 7,5 | 0,048 |
| ABG | 0,56 | 7,5 | 0,048 |
| MLE | 0,59 | 8 | 0,076 |
| MLO | 0,51 | 5 | 0,076 |
| EMA | 0,40 | 8 | 0,076 |
| MLA | 0,48 | 8 | 0,076 |
| DMA | 0,48 | 8 | 0,076 |
| DLM | 0,50 | 8 | 0,076 |
| IDA | 0,74 | 8 | 0,132 |
| KJA | 0,89 | 8 | 0,16 |
| TPL | 0,63 | 5,5 | 0,132 |
| TIR | 0,67 | 8 | 0,104 |

Au vu des simulations de répartition du magnésium neutre en fonction du pH, il a paru intéressant de simuler la répartition des espèces magnésiennes neutres formées avec un mélange de deux ligands.

En effet, lorsque les espèces neutres de deux systèmes existent dans des zones de pH différentes, on a cherché à obtenir la fraction de magnésium neutre sur une plus grande zone de pH. Pour ce faire, les mélanges de ligands et les concentrations utilisées ont été choisis d'après les résultats des simulations propres à chaque ligand. Les vecteurs qui se sont avérés les plus intéressants sont les ligands qui donnent naissance à une espèce magnésienne neutre de stabilité relativement importante dans la zone de pH 6-7 : MLA, MLO, MLE.

Les résultats des simulations sont rapportés dans les Tableaux 6, donnant pour chaque mélange envisagé, les pourcentages d'espèce neutre aux pH 5, 7 et 8 pour divers rapports de concentrations en ligands. Pour comparaison, la première série de tableaux (6.A) donne les pourcentages relatifs aux ligands seuls. La concentration en magnésium est 0,05 mol.dm-3. La concentration en ligands est donnée à la première ligne des tableaux : 0,05 ou 0,1 mol.dm-3.

13

Tableaux 6

| 6.A | | | | | | |
|---|---|---|---|---|---|---|
| | MLE | MLE | MLA | MLA | MLO | MLO |
| | 0,05 | 0.1 | 0.05 | 0.1 | 0.05 | 0.1 |
| pH 5: | 13 | 21 | 34 | 48 | 44 | 51 |
| pH 7: | 49 | 59 | 42 | 47 | 49 | 45 |
| pH 8: | 50 | 59 | 42 | 47 | 41 | 38 |
| 6.B.a b | | | | | | |
| | MLE MLA | MLE MLA | MLE MLO | MLE MLO | | |
| | 0.05 0.05 | 0.1 0.05 | 0.05 0,05 | 0,1 0.05 | | |
| pH 5 | 40 | 39 | 42 | 41 | | |
| pH 7 | 63 | 62 | 62 | 59 | | |
| pH 8 | 63 | 62 | 57 | 55 | | |

Les mélanges MLE MLA (6.B.a) et MLE MLO (6.B.b) conduisent à l'obtention d'espèces neutres dont le pourcentage est très supérieur à celui de chaque ligand pris séparément : pour le rapport Mg MLE MLA égal à 1 1 11, on obtient à pH 5. 7 et 8, respectivement 40, 63 et 63 % à comparer à 13, 49 et 50 % pour MLE et 34, 42 et 42 % pour MLA ; le rapport 1 2 1 est également très favorable. Le cas est sensiblement identique pour MLE MLO.

Enfin, l'influence de concentrations croissantes de chacun des ligands ci-dessus sur la répartition de la fraction ultrafiltrable (non liée aux protéines) du magnésium plasmatique a été simulée au moyen du programme ECCLES pour une concentration libre d'ions Mg2 + égale à 0.520.10-3 mol.dm-3 et à pH = 7,4.

Parmi les meilleurs résultats on trouve. outre le malonate (43.6% de NMg pour CLigand = 1.10-2 mol.dm-3), le maléate (35% de NMg) et le malate (30% de NMg). On constate que ces ligands sont capables de mobiliser une fraction importante de magnésium ultrafiltrable du plasma sous forme neutre.

III - Acide malique

Les études précédentes ont permis de sélectionner par simulation l'acide malique comme vecteur susceptible d'optimiser l'absorption du magnésium par un organisme humain ou animal. Par ailleurs, ce composé apparait particulièrement intéressant en matière pharmaceutique et diététique pour les raisons suivantes : il est ingérable (abondant dans la pomme et d'autres fruits) et dénué de toxicité. De plus, sa forme (DL)-malique est d'un coût de fabrication raisonnable.

L'acide malique existe sous la forme de deux isomères optiques : le (L)-malique (MLA), produit initialement étudié. et le (D)-malique (DMA). Il existe aussi le (DL)-malique (DLM), mélange racémique des formes D et L. Il a donc été nécessaire de s'assurer que les propriétés chimiques de ces composés étaient identiques à celles du (L)-malique.

Une étude systématique selon la méthode précédemment exposée a été effectuée sur l'acide (D)-malique. l'acide racémique (DL)-malique de qualité pour analyses et l'acide (DL)-malique provenant de l'usine GIVAUDAN-LAVIROTTE.

Les constantes de protonation de ces produits et les constantes de formation de leurs complexes de magnésium sont respectivement rapportées dans les Tableaux 3 et 4 présentés ci-avant. Les valeurs obtenues pour tous ces produits sont très voisines et les simulations de la répartition des complexes du magnésium en fonction du pH montrent que le pourcentage d'espèce neutre ML est sensiblement identique pour tous :

- pour une concentration de 0.005 mol.dm-3 en magnésium et de 0,05 mol.dm-3 en ligand, il existe 50% d'espèce neutre ML de pH 5 à 8 avec 30% d'espèce ML2 (figure 22 illustrant la simulation pour l'acide (DL)-malique d'usine).

- pour une concentration en magnésium et ligand égale à 0,05 mol.dm-3, il se forme 44% d'espèce ML et 10% d'espèce ML2 (figure 23 - acide (DL)-malique d'usine). Pour les conditions de concentration du fluide gastro-intestinal. la simulation calculée avec le programme TRIPLOT montre que le pourcentage d'espèce neutre est maximum (50% à pH 8) lorsque, pour une concentration en magnésium de 0,04 mol.dm-3, la concentration en ligand est de 0,076 mol.dm-3. En présence d'une concentration en calcium égale à celle de magnésium (0,04 mol.dm-3), on obtient le même pourcentage pour une concentration en ligand égale à 0,132 mol.dm-3.

Par ailleurs, la simulation des variations de la concentration du malate dans le plasma sanguin à partir de la concentration moyenne physiologique normale de celui-ci (3,5.10-5 mol.dm-3) révèle un accroissement très significatif du pourcentage de complexe neutre ML (à ligand malate) en fonction de l'augmentation de la concentration du malate CMLA. Ces résultats sont illustrés dans le Tableau 7 ci-dessous dans lequel :

% Mg : représente le pourcentage de la fraction de Mg sous forme de métal libre par rapport à la fraction ultra filtrable disponible dans le plasma.

% NMg : représente le pourcentage de la fraction de la totalité de complexes neutres de l'ion Mg2 + (NMg) par rapport à la fraction ultra filtrable disponible.

% ML : représente le pourcentage de la fraction du complexe neutre "malate Mg2 + " (ML) par rapport à la fraction ultra filtrable disponible.

% ML NMg : représente en % le rapport de la fraction de complexes neutres "malate Mg2 + " (ML) sur la fraction de la totalité des complexes neutres de l'ion Mg2 + (NMg).

Tableau 7

| CMLA (mol.dm-3) | % Mg | % NMg | dont | % ML | % ML NMg |
|---|---|---|---|---|---|
| 3,5.10-5 | 75,4 | 7,9 | | 0,1 | 1,3 |
| 1,1.10-4 | 75,1 | 8,1 | | 0,4 | 4,9 |
| 3,5.10-4 | 74,5 | 8,9 | | 1,2 | 13,5 |
| 1,1.10-3 | 72,5 | 11,2 | | 3,7 | 33,0 |
| 3,5.10-3 | 66,7 | 17,5 | | 10,7 | 61,1 |
| 1,1.10-2 | 52,7 | 30,8 | | 25,5 | 82,8 |
| 3,5.10-2 | 30,5 | 44,6 | | 41,5 | 93,0 |

Il résulte de ce qui précède que le malate est également susceptible de favoriser sensiblement l'absorption tissulaire du magnésium à partir du plasma par l'augmentation importante de la concentration de complexe neutre "malate Mg2 + " dans le plasma résultant du transfert de l'ion malate à partir du fluide gastro-intestinal soit sous la forme neutre complexée avec le magnésium, soit sous la forme neutre protonée (acide non dissocié).

Ainsi donc l'utilisation de l'acide (DL)-malique dans les compositions pharmaceutiques et diététiques apparait tout à fait recommandée. Cette conclusion a été confirmée par une série d'expérimentations in vivo sur des rats mettant en jeu l'acide (DL)-malique et rapportées ci-après.

Expérimentation sur le rat.

A - 1. Protocole expérimental.

L'essai porte sur 60 rats mâles S.P.F. de souche "CD", d'un poids moyen de 60 à 80 g à leur arrivée. Ils sont maintenus en animalerie conventionnelle dans des conditions identiques de température (21°C + - 1°C), d'hygrométrie (40 à 70 %) et de luminosité (12h/24).

Dès leur arrivée, les animaux sont placés en cage à métabolisme et reçoivent un aliment contenant 570 mg de Mg2 + /kg : concentration nécessaire et suffisante pour maintenir chez l'animal une magnésémie normale.

4 jours après leur arrivée, au temps "t-16h" de l'essai, soit 16 h avant l'administration des traitements (temps "t"), les animaux sont pesés, mis à jeun et le volume d'eau déminéralisée mis à leur disposition est exactement mesuré.

Au temps "t-1h" soit 1 h avant l'administration des traitements, l'eau de boisson est retirée. Elle est redistribuée 30 minutes après cette administration, le volume étant exactement mesuré.

15

Au temps "t + 24h" les animaux sont sacrifiés par dislocation cervicale et le contenu de la vessie et des intestins est prélevé.

Les animaux sont répartis en 3 lots de 20 animaux chacun :

. Lot I : Témoins recevant 40 ml.kg d'eau déminéralisée;

. Lot II : Traités recevant 38,88 mg de Mg2 + kg sous forme de MgCl2, sous un volume de 40 ml.kg d'eau déminéralisée ;

. Lot III : Traités recevant l'association "MgCl2 + acide malique" à la dose de 38,88 mg de Mg2 + kg sous forme de MgCl2 + 408 mg d'acide malique.kg, sous un volume de 40 ml.kg d'eau déminéralisée (pH ajusté à 5). ce qui correspond à une concentration en magnésium CMg = 0,04 mol.dm-3 et à une concentration de (DL)-malate CDLM = 0,076 mol.dm-3.

A - 2. Examens.

a. - Avant traitement : "t - 16 h" à "t"

. Recueil des urines et mesure du volume.

. Mesure de la consommation hydrique 15 h (les animaux étant privés d'eau de boisson 1 h avant l'administration des traitements).

b. - Traitement : "t"

. Pesée des animaux.

. Administration des traitements. voie orale, par tubage oesophagien.

c. - Après traitement : "t" à "t + 24 h"

c.1. Exploration urinaire :

. Recueil des urines de "t à t + 1 h". de "t + 1 h" à "t + 4 h", de "t + 4 h à t + 8 h" et de "t + 8 h à t + 24 h" après administration des traitements ; l'urine contenue dans la vessie étant ajoutées au recueil de "t + 8 h à t + 24 h".

. Mesure des différents volumes et dosage du Mg2 + sur chaque fraction.

c.2. Exploration au niveau fécal :

. Recueil des fèces émises pendant 24 h auxquelles sont ajoutées, à "t + 24 h", les fèces contenues dans les intestins.

. Pesée des fèces et dosage de Mg2 + sur la totalité des fèces.

c.3. Mesure de la consommation hydrique .

Du point de vue dosage. la méthode colorimétrique à la calmagite a été utilisée pour le magnésium urinaire et pour le magnésium fécal (après calcination de la totalité des fèces recueillies).

A - 3. Résultats :

Pour les urines. les volumes sont exprimés en ml/100 g de rat (V.U) et la concentration de magnésium est exprimée en mg. 100 g de rat (MgU). Le résultat exprimé par 24 h (par ex : V.U. 24 h) est la somme des résultats obtenus à chaque période de recueil.

Pour les fèces, la concentration est exprimée en mg. 100 g de rat (MgF).

Les coefficients du bilan magnésique sont calculés pour les lots II et III d'après les formules suivantes dans lesquelles :

MgI = Magnésium Ingéré,

MgF tr. = Magnésium Fécal de l'animal traité.

MFM té. = Magnésium Fécal Moyen des animaux témoins.

MgU tr. = Magnésium Urinaire de l'animal traité.

MUM té. = Magnésium Urinaire Moyen des animaux témoins.

. Coefficient d'utilisation digestive (C.u.d.) :

$$C.u.d. = \frac{MgI-(MgF\ tr. - MFM\ té.)}{MgI} \times 100$$

Ce coefficient met en évidence la faculté de passage de la membrane gastro-intestinale pour le surplus de magnésium ingéré (MgI) à partir de l'exploitation de la variation du magnésium recueilli dans les fèces entre le sujet traité et la valeur moyenne témoin (absorption gastro-intestinale).
. Coefficient de rétention (C.r.) :

$$C.r. = \frac{MgI-((MgF\ tr.- MFM\ té.)+(MgU\ tr.\ -\ MUM\ té.))}{MgI\ -\ (MgF\ tr.\ -\ MFM\ té.)} \times 100$$

Ce coefficient met en évidence la faculté de passage dans les tissus pour le surplus de magnésium ingéré ayant déjà traversé la membrane gastro-intestinale à partir de l'exploitation de la variation du magnésium recueilli dans les urines entre le sujet traité et la valeur moyenne témoin (absorption tissulaire).
. Coefficient d'absorption (C. abs.) :

$$C.\ abs. = \frac{MgI-((MgF\ tr.- MFM\ té.)+(MgU\ tr.-MUM\ té))}{MgI} \times 100$$

Ce coefficient met en évidence la faculté d'absorption globale du magnésium ingéré dans les tissus, mettant en jeu les deux mécanismes d'absorption gastro-intestinal et tissulaire.

Pour tous les résultats donnés dans les tableaux 8 et 9 ci-après, le degré de signification des différences traitement "MgCl2" traitement "MgCl2 + acide malique" est déterminé par le test "t - indépendant" de Student.

## Tableau 8

| LOT | | V.U/24 h | MgU/24 h | MgF/24 h |
|-----|-----|-----|-----|-----|
| I | M | 10,36 | 0,568 | 0,626 |
| | E.T | 3,68 | 0,207 | 0,318 |
| II | M | 11,70 | 1,916 | 1,652 |
| | E.T | 4,44 | 0,395 | 0,636 |
| III | M | 9,59 | 1,241 | 1,805 |
| | E.T | 2,38 | 0,296 | 0,620 |
| | S (II) | NS | S*** | NS |

Où M = Moyenne, E.T = Ecart-type et S (II) = Signification de la comparaison par rapport au lot II avec:

NS = différence non significative P>0,05

S* = différence significative P<0,05

S** = différence significative P<0,01

S*** = différence significative P<0,001

Tableau 9

| LOT | | C.u.d. | C.r. | C. abs. |
|---|---|---|---|---|
| II | M | 73,617 | 50,083 | 38,948 |
| | E.T | 16,360 | 19,717 | 20,372 |
| III | M | 69,683 | 74,516 | 51,986 |
| | E.T | 15,946 | 10,313 | 14,159 |
| | S (II) | NS | S''' | S' |

L'examen du Tableau 8 montre :

. Des volumes urinaires sur 24 h comparables (Il a toutefois été remarqué une réduction sensible du volume urinaire chez les sujets du lot III pendant la période "t" à "t + 1 h").

. Des excrétions de magnésium urinaire plus faibles de façon très significatives pour le lot III.

. Des excrétions de magnésium fécal comparables pour les lots II et III.

L'examen du Tableau 9 montre un coefficient d'utilisation digestive comparable pour les deux lots traités, tandis que nous observons une augmentation du coefficient de rétention et d'absorption de $Mg2+$ chez les animaux recevant l'association "MgCl2 + acide malique" par rapport aux traités recevant MgCl2 seul.

En résumé. la quantité de $Mg2+$ fécale excrétée n'est pas significativement différente entre les deux traitements : MgCl2 et association "MgCl2 + acide malique".

L'absorption gastro-intestinale est ici comparable dans les deux lots.

Par contre, l'excrétion urinaire s'est révélée nettement plus faible chez les animaux recevant l'association "MgCl2 + acide malique". ce qui signifierait une augmentation de la diffusion tissulaire du $Mg2+$ à partir du plasma. Les coefficients de rétention et d'absorption plus élevés chez ces animaux confirmeraient cette hypothèse.

A - 4. Résultats complémentaires.

D'autres expérimentations basées sur des protocoles comparables ont confirmé les résultats ci-dessus. Elles ne feront pas toutefois l'objet d'une présentation en détails. Seuls les résultats de l'expérimentation suivante mettant en jeu l'acide malique et l'acide citrique seront présentés de façon globale dans les tableaux 10 et 11 ci-après.

La nouvelle expérimentation (codée 1.33) a été réalisée sur 4 lots de 18 animaux chacun. les lots I, II et III recevant les mêmes traitements que ceux administrés aux lots correspondants dans le cadre de l'expérimentation précédente et le lot IV recevant l'association "MgCl2 + acide citrique" à la dose de 38,88 mg de $Mg2+$ kg sous forme de MgCl2 + 1345 mg d'acide citrique/kg, sous un volume de 40 ml kg d'eau déminéralisée (pH ajusté à 5), ce qui correspond à une concentration en magnésium CMg = 0,04 mol.dm-3 et à une concentration de citrate CTA = 0,16 mol.dm-3.

Tableau 10

| LOT | | V.U/24 h | MgU/24 h | MgF/24 h |
|---|---|---|---|---|
| I | M | 9,86 | 0,446 | 0,715 |
| | E.T | 6,43 | 0,167 | 0,338 |
| II | M | 9,45 | 1,873 | 2,003 |
| | E.T | 3,65 | 0,506 | 0,566 |
| III | M | 8,97 | 1,573 | 1,851 |
| | E.T | 3,85 | 0,567 | 0,675 |
| | S (II) | NS | NS | NS |
| IV | M | 8,43 | 1,233 | 1,958 |
| | E.T | 3,35 | 0,419 | 0,593 |
| | S (II) | NS | S*** | NS |

Tableau 11

| LOT | | C.u.d. | C.r. | C. abs. |
|---|---|---|---|---|
| II | M | 66,89 | 43,78 | 30,19 |
| | E.T | 14,56 | 19,62 | 16,24 |
| III | M | 70,79 | 58,87 | 41,81 |
| | E.T | 17,35 | 19,94 | 17,40 |
| | S (II) | NS | S* | S* |
| IV | M | 68,03 | 68.08 | 47,81 |
| | E.T | 15,25 | 23,54 | 16,79 |
| | S (II) | NS | S*** | S*** |

Ainsi donc, les divers essais présentés dans cet exposé mettant en jeu l'acide (DL)-malique ont tous permis de vérifier l'amélioration de l'absorption du magnésium par un organisme animal, notamment au niveau de la rétention tissulaire. Il est à remarquer que l'utilisation de l'acide citrique donne aussi de bon résultats au niveau de la rétention tissulaire.

IV - Applications pharmaceutiques et diététiques :

Tous les ligands de l'ion Mg2 + pour former avec celui-ci des complexes électriquement neutres présentés ci-avant et administrables dans un organisme humain ou animal, sont susceptibles d'être utilisés comme constituants d'agents-vecteurs favorisant l'absorption du magnésium dans cet organisme. Ils sont susceptibles d'être incorporés dans des compositions pharmaceutiques utilisables en magnésothérapie humaine ou vétérinaire et/ou dans des préparations diététiques pour des supplémentations en magnésium dans le cadre de régimes diététiques humain ou animal tout en respectant les domaines de concentration en ligand imposés par les résultats des études de toxicité conformes à la pratique courante dans la profession.

En particulier, sont concernés par l'invention les agents-vecteurs du type comportant un ou plusieurs ligands de l'ion magnésium Mg2 + pour former au moins un complexe électriquement neutre et administrable(s) dans un organisme humain ou animal et présentant, pour être associé dans une solution monométallique à un ou plusieurs sels de magnésium, une concentration en ligand(s) adaptée à la concentration choisie en ion Mg2 + pour assurer un pourcentage d'ions magnésium Mg2 + sous forme de complexe électriquement neutre au moins égal à 10 % en concentration molaire pour un pH compris entre 6,5 et 8.

De plus, entrent dans le cadre de l'invention les compositions pharmaceutiques et diététiques contenant du magnésium en tant que seul élément métallique actif et au moins un agent-vecteur de type défini ci-dessus.

Parmi d'autres modes de réalisation de l'invention, appartiennent les agents-vecteurs spécifiés ci-dessous :

- Agent-vecteur du type comportant un ou plusieurs composés suivants ou leurs sels d'addition administrables dans un organisme humain ou animal, et agissant en tant qu'apporteur de ligand(s) de l'ion $Mg2+$ : acide maléique, acide malonique et acide acide malique.

- Agent-vecteur du type comportant au moins deux des composés suivants ou leurs sels d'addition administrables dans l'organisme : acide malique, acide maléique, acide malonique et acide citrique.

D'autres compositions pharmaceutiques ou diététiques entrant dans le cadre de l'invention sont constituées à partir d'un ou plusieurs agents-vecteurs ci-dessus spécifiés en association ou non avec un autre composé ou sel apporteur de magnésium (pour former préalablement ou postérieurement à leurs administrations au moins un complexe électriquement neutre avec l'ion $Mg2+$) et ou à partir des complexes électriquement neutres de l'ion $Mg2+$ du type MqLp et ou du type MqLpHr.

A titre d'exemple non limitatif, l'apport en magnésium est réalisé par des composés ioniques facilement dissociables en solution aqueuse et susceptibles de libérer l'ion $Mg2+$, et se présentant soit sous la forme de chlorure de magnésium ,soit sous la forme de carbonate de magnésium additionné de soude afin de stabiliser la solution administrable autour d'un pH compris entre 4 et 5 pour le confort du sujet-utilisateur. Dans ce dernier cas, grâce à l'élimination du bioxyde de carbone sous forme gazeuse, la quantité nécessaire d'agent neutralisant reste faible ce qui diminue d'autant la concentration en ion $Na+$ dans la solution administrable (l'ion sodium n'agissant pas à titre d'élément métallique actif de la composition ainsi préparée). Cette dernière composition est recommandée pour des sujets soumis à un régime sans sodium.

L'invention concerne également une composition pharmaceutique ou diététique destinée à la magnésothérapie humaine ou vétérinaire et ou aux supplémentations magnésiennes, la composition contenant en quantité efficace deux ingrédients actifs, distincts ou non, de l'acide citrique ou un de ses sels administrables dans l'organisme et un composé apporteur de l'ion $Mg2+$, en association avec un excipient ou un véhicule pharmaceutique acceptable.

Préférentiellement les compositions selon l'invention sont conditionnées sous forme de préparations administrables dans l'organisme par voie orale ou parentérale. En ce qui concerne l'administration par voie orale, les compositions pharmaceutiques et diététiques selon l'invention peuvent apparaître sous forme de solutions aqueuses prépréparées, éventuellement à diluer, sous forme de compositions à dissoudre dans un verre d'eau et sous forme de comprimés ou de tablettes à sucer ou à croquer.

Un exemple de solution préparée pour 100 cm3 est donné dans la formule N°1 ci-dessous :

| FORMULE N°1 POUR UNE DOSE | | |
|---|---|---|
| Acide (DL)-malique : | 1,05 g | # 7,83 mmol |
| Magnésium (MgCl2) : | 0,10 g | # 4,11 mmol |
| NaOH qsp pH 5 : | 0,28 g | # 7,0 mmol |
| Saccharose : | 2,00 g | |
| Jus de fruit : | 40 cm3 | |
| Eau qsp : | 100 cm3 | |
| pH final    = | 4,5 | |

Un autre exemple de solution buvable présentée sous forme d'ampoule et comportant également du chlorure de magnésium (MgCl2) est donné dans la formule N°2 ci-dessous:

| FORMULE N°2 POUR UNE DOSE | | |
|---|---|---|
| Acide (DL)-malique : | 1,05 g | # 7,83 mmol |
| Magnésium (MgCl2) : | 0,10 g | # 4,11 mmol |
| Soude (NaOH) ou autre composé alcalinisant : | qsp pH 5 | |
| Excipient et eau qsp: | 10 cm3 | |

Deux exemples de compositions de tablettes à sucer sont donnés dans dans les formules N°3 et N°4 ci-dessous:

| FORMULE N°3 POUR UNE TABLETTE | | |
|---|---|---|
| Acide (DL)-malique : | 1,05 g | # 7,83 mmol |
| Magnésium (MgCl2) : | 0,10 g | # 4,11 mmol |
| Excipient qsp : | une tablette | |

| FORMULE N°4 POUR UNE TABLETTE | | |
|---|---|---|
| Acide citrique : (monohydraté) | 1,05 g | # 5,0 mmol |
| Magnésium (MgCl2) : | 0,10 g | # 4,11 mmol |
| Excipient qsp: | une tablette | |

Il est à noter par ailleurs que l'on a retenu de préférence pour la présentation par voie orale une dose d'au moins 25 mg par prise (par exemple sous forme de chlorure de magnésium) avec un rapport de concentration molaire ligand/magnésium de l'ordre de 2 pour le malate et compris entre 1,2 et 4 pour le citrate (le rapport citrate/magnésium étant de 0,75 pour le sel stoechiométrique de citrate de magnésium).

Bien entendu les formules ci-dessus sont données à titre d'exemples non limitatifs et peuvent être modifiées sans sortir du cadre de l'invention pour faciliter la fabrication et ou pour des raisons d'acceptation par les utilisateurs. Par exemple, à titre de variantes de l'invention, les compositions sont basées sur un mélange des trois ingrédients actifs acide malique, acide citrique et chlorure de magnésium.

En ce qui concerne l'administration en solutions diluées, les concentrations optimales du ligand CL sont données à partir du Tableau 5 pour les divers ligands étudiés pour un apport d'ion Mg2 + de 100 mg dans 100 cm3 soit CMg = 0,04 mol.dm-3 (apport correspondant environ au tiers du besoin journalier en magnésium d'un sujet humain adulte, 300 mg/j). Pour le ligand (DL)-malate, cette concentration optimale CL est de 0,076 mol.dm-3 pour CMg égal à 0, 04 mol.dm-3.

En ce qui concerne les intervalles de concentration recommandés pour ces ligands, il faut remarquer que les concentrations maximales sont imposées par des limites de solubilité et/ou de toxicité des produits. Par contre, les concentrations minimales de ligand ont été obtenues par simulation comme correspondant à au moins 10% en concentration molaire d'ions Mg2 + complexés sous forme neutre et pour la dose minimum de magnésium considérée comme efficace soit 25 mg pour 200 cm3 maximum (CMg = 0,005 mol.dm-3).

Ces concentrations minimum sont données en mol.dm-3 dans le Tableau 12 ci-après :

Tableau 12

| Vecteur | Concentration minimum |
|---------|----------------------|
| Citrate | 0,0050 |
| Maléate | 0,0021 |
| Malonate | 0,0017 |
| L-Malate | 0.0020 |
| D-Malate | 0,0020 |
| DL-Malate | 0,0020 |

Par ailleurs. mais toujours dans le cadre de l'invention, de nouveaux complexes relativement stables de l'ion Mg2 + ont été présentés et mis en évidence expérimentalement, plus particulièrement les complexes, notamment ceux électriquement neutres, du type MqLp ou du type MqLpHr où M représente l'ion Mg2 + , H représente le proton et L représente un ligand choisi dans le groupe suivant : maléate, malonate et malate (L-malate, D-malate. DL-malate). Il est entendu que l'utilisation de ces complexes comme constituant de médicament. en particulier de médicament applicable à la magnésothérapie humaine et vétérinaire et ou comme constituant de préparations diététiques destinées aux supplémentations dans le cadre de régimes diététiques humain ou animal, entre dans le cadre de l'invention.

L'invention concerne également les méthodes d'obtention des agents-vecteurs. des compositions de pharmacie ou de diététique et des complexes de l'ion magnésium Mg2 + présentés ci-dessus. en application des enseignements de la présente description, de même que l'utilisation des agents-vecteurs, compositions et complexes ainsi obtenus pour faciliter l'absorption du magnésium dans un organisme humain ou animal.

## Revendications

1. Agent-vecteur facilitant l'absorption d'une dose efficace de magnésium dans un organisme humain ou animal, en tant que seul élément métallique actif, caractérisé en ce qu'il comporte un ou plusieurs ligands de l'ion magnésium Mg2 + pour former au moins un complexe électriquement neutre administrable-(s) dans l'organisme et en ce qu'il présente, pour être associé dans une solution monométallique à un ou plusieurs sels de magnésium. une concentration en ligand(s) adaptée à la concentration choisie en ion Mg2 + pour assurer un pourcentage d'ions magnésium sous forme de complexe électriquement neutre au moins égal à 10% en concentration molaire pour un pH compris entre 6.5 et 8.

2. Composition de pharmacie ou de diététique humaine ou vétérinaire. caractérisée en ce qu'elle contient du magnésium en tant que seul élément métallique actif et comporte au moins un agent-vecteur selon la revendication précédente.

3. Composition selon la revendication 2. caractérisée en ce que l'agent-vecteur est associé à un composé ionique administrable dans un organisme humain ou animal, facilement dissociable en solution aqueuse et susceptible de libérer l'ion Mg2 + en vue de sa complexation. notamment du chlorure de magnésium ou du carbonate de magnésium.

4. Composition selon la revendication 3. caractérisée en ce que l'agent-vecteur est associé à du carbonate de magnésium accompagné éventuellement d'un composé neutralisant en quantité suffisante pour stabiliser entre 4 et 5 le pH de la solution administrable correspondante.

5. Composition selon l'une des revendications 2 à 4, caractérisée en ce qu'elle comporte au moins 25 mg d'élément métallique magnésium, administrables en solution aqueuse et correspondants à une concentration minimum de 0.005 mol.dm-3, associés à un agent-vecteur dont la concentration assure un pourcentage d'ions magnésium sous forme de complexe électriquement neutre au moins égal à 10 % en concentration molaire.

6. Utilisation d'un agent-vecteur selon la revendication 1 et ou d'une composition selon l'une des revendications 2 à 5 à la magnésothérapie humaine ou vétérinaire et ou aux supplémentations magnésiennes dans le cadre de régimes diététiques humain et animal.

7. Agent-vecteur facilitant l'absorption du magnésium dans un organisme humain ou animal et destiné à être administré soit en tant que seul apporteur de magnésium, soit en association avec un autre composé apporteur de magnésium, caractérisé en ce qu'il contient un ou plusieurs des composés suivants ou de

22

leurs sels d'addition administrables dans l'organisme, et agissant en tant qu'apporteur de ligand de l'ion Mg2+: acide maléique, acide malonique et acide malique.

8. Agent-vecteur selon la revendication 7, caractérisé en ce qu'il contient au moins deux des composés suivants ou leurs sels d'addition administrables dans l'organisme : acide maléique, acide malonique, acide malique et acide citrique.

9. Composition de pharmacie ou de diététique humaine ou vétérinaire, caractérisée en ce qu'elle comporte au moins un agent-vecteur selon l'une des revendications 7 et 8, éventuellement associé avec un autre composé ou sel apporteur en magnésium pour former préalablement ou postérieurement à son administration avec l'ion Mg2+ au moins un complexe électriquement neutre, notamment le chlorure de magnésium ou le carbonate de magnésium.

10. Composition selon la revendication 9, caractérisée en ce que l'agent-vecteur est associé à du carbonate de magnésium éventuellement accompagné d'un composé neutralisant en quantité suffisante pour stabiliser entre 4 et 5 le pH de la solution administrable correspondante.

11. Composition selon l'une des revendications 9 et 10, caractérisée en ce qu'elle comporte au moins 25 mg d'élément métallique magnésium, administrables en solution aqueuse de concentration minimum de 0,005 mol.dm-3, associés à un agent-vecteur dont la concentration minimum correspondante exprimée en mol.dm-3 est définie dans le tableau ci-après :

| Agent-vecteur | Concentration minimum |
|---|---|
| Maléate | 0,0021 |
| Malonate | 0,0017 |
| Malate | 0,0020 |

12. Composition selon l'une des revendications 9 à 11, caractérisée en ce que l'agent-vecteur contient de l'acide (DL)-malique ou un de ses sels administrables dans l'organisme, la concentration en ligand (DL)-malate étant de préférence égale à 0,076 mol.dm-3 pour une concentration de magnésium égale à 0,04 mol.dm-3.

13. Composition selon la revendication 9, caractérisée en ce que l'agent-vecteur contient de l'acide (DL) - malique ou un de ses sels administrables dans l'organisme, le rapport des concentrations molaires ligand-malate magnésium étant de préférence de l'ordre de 2.

14. Composition pharmaceutique ou diététique destinée à la magnésothérapie humaine ou vétérinaire et ou aux supplémentations magnésiennes, caractérisée en ce qu'elle contient en quantité efficace deux ingrédients actifs, distincts ou non, de l'acide citrique ou un de ses sels administrables dans l'organisme et un composé apporteur de l'ion Mg2+, en association avec un excipient ou un véhicule pharmaceutique acceptable.

15. Composition de pharmacie ou de diététique humaine ou vétérinaire selon la revendication 14, caractérisée en ce qu'elle comporte l'ion citrate à titre de ligand d'au moins 25 mg d'ion Mg2+ administrables en solution aqueuse et correspondants à une concentration minimum de 0,005 mol.dm-3, pour former un complexe électriquement neutre de l'ion Mg2+, selon une concentration minimale de 0,005 mol.dm-3 pour l'ion citrate seul, la concentration citrate du ligand seul étant de préférence égale à 0,16 mol.dm-3 pour une concentration d'ion Mg2+ égale à 0,04 mol.dm-3.

16. Composition de pharmacie ou de diététique humaine ou vétérinaire selon la revendication 14, caractérisée en ce qu'elle contient de l'acide citrique ou un de ses sels administrables dans l'organisme pour agir en tant que ligand d'au moins 25 mg d'ion Mg2+, le rapport des concentrations molaires ligand-citrate magnésium étant de préférence compris entre 1,2 et 4.

17. Composition selon l'une des revendications 9 à 16, caractérisée en ce qu'elle comporte du carbonate de magnésium accompagné éventuellement d'un composé neutralisant en quantité suffisante pour stabiliser entre 4 et 5 le pH de la solution administrable correspondante.

18. Composition selon l'une des revendications 2 à 5 et 9 à 17, caractérisée en ce qu'elle est conditionnée sous forme de préparation administrable dans l'organisme par voie orale ou par voie parentérale.

19. Complexes, notamment complexes électriquement neutres, de l'ion magnésium Mg2+ du type MqLp et/ou du type MqLpHr où M représente l'ion Mg2+, H représente le proton et L représente un ligand choisi dans le groupe suivant: maléate, malonate et malate.

20. Utilisation d'un agent-vecteur selon l'une des revendications 7 et 8 et/ou d'une composition selon l'une des revendications 9 à 18 et ou d'un complexe ingérable selon la revendication 19 à la magnésothérapie humaine ou vétérinaire et ou aux supplémentations magnésiennes dans le cadre de régimes diététiques humain et animal.

Revendications pour l'Etat contractant suivant: ES

1. Procédé pour obtenir un agent-vecteur et/ou une composition facilitant l'absorption d'une dose efficace de magnésium dans un organisme humain ou animal, en tant que seul élément métallique actif, caractérisé en ce que l'on associe dans une solution monométallique à un ou plusieurs sels de magnésium un ou plusieurs ligands de l'ion magnésium Mg2+ à titre d'agent-vecteur pour former au moins un complexe électriquement neutre administrable(s) dans l'organisme, la concentration en ligand(s) étant adaptée à la concentration choisie en ion Mg2+ pour assurer un pourcentage d'ions magnésium sous forme de complexe électriquement neutre au moins égal à 10% en concentration molaire pour un pH compris entre 6.5 et 8.

2. Procédé selon la revendication 1, caractérisé en ce que l'agent-vecteur est associé à un composé ionique administrable dans un organisme humain ou animal, facilement dissociable en solution aqueuse et susceptible de libérer l'ion Mg2+ en vue de sa complexation, notamment du chlorure de magnésium ou du carbonate de magnésium.

3. Procédé selon la revendication 2, caractérisé en ce que l'agent-vecteur est associé à du carbonate de magnésium accompagné éventuellement d'un composé neutralisant en quantité suffisante pour stabiliser entre 4 et 5 le pH de la solution administrable correspondante.

4. Procédé selon l'une des revendications 1 à 3, caractérisé en ce que l'on associe au moins 25 mg d'élément métallique magnésium, administrables en solution aqueuse et correspondants à une concentration minimum de 0.005 mol.dm-3, à un agent-vecteur dont la concentration assure un pourcentage d'ions magnésium sous forme de complexe électriquement neutre au moins égal à 10 % en concentration molaire.

5. Utilisation d'un agent-vecteur et ou d'une composition obtenu selon l'une des revendications précédentes à l'administration de magnésium dans un organisme humain ou animal.

6. Utilisation à la préparation d'une composition destinée à l'administration de magnésium dans un organisme humain ou animal, d'un agent-vecteur facilitant l'absorption du magnésium dans un organisme humain ou animal et destiné à être administré soit en tant que seul apporteur de magnésium, soit en association avec un autre composé apporteur de magnésium, ledit agent-vecteur contenant un ou plusieurs des composés suivants ou de leurs sels d'addition administrables dans l'organisme, et agissant en tant qu'apporteur de ligand de l'ion Mg2+ : acide maléique, acide malonique et acide malique.

7. Utilisation selon la revendication 6, caractérisé en ce que l'agent-vecteur contient au moins deux des composés suivants ou leurs sels d'addition administrables dans l'organisme: acide maléique, acide malonique, acide malique et acide citrique.

8. Utilisation selon l'une des revendications 6 et 7, caractérisée en ce que l'agent-vecteur est associé avec un autre composé ou sel apporteur de magnésium pour former préalablement ou postérieurement à son administration avec l'ion Mg2+ au moins un complexe électriquement neutre, notamment le chlorure de magnésium ou le carbonate de magnésium.

9. Utilisation selon la revendication 8, caractérisée en ce que l'agent-vecteur est associé à du carbonate de magnésium éventuellement accompagné d'un composé neutralisant en quantité suffisante pour stabiliser entre 4 et 5 le pH de la solution administrable correspondante.

10. Procédé pour obtenir une composition destinée à l'administration de magnésium dans un organisme humain ou animal, caractérisé en ce que l'on associe au moins 25 mg d'élément métallique magnésium, administrables en solution aqueuse de concentration minimum de 0,005 mol.dm-3, à un agent-vecteur dont la concentration minimum correspondante exprimée en mol.dm-3 est définie dans le tableau ci-après :

| Agent-vecteur | Concentration minimum |
|---|---|
| Maléate | 0,0021 |
| Malonate | 0,0017 |
| Malate | 0,0020 |

11. Procédé selon la revendication 10, caractérisé en ce que l'agent-vecteur contient de l'acide (DL)-malique ou un de ses sels administrables dans l'organisme, la concentration en ligand (DL)-malate étant de préférence égale à 0,076 mol.dm-3 pour une concentration de magnésium égale à 0,04 mol.dm-3.

12. Procédé pour obtenir une composition destinée à l'administration de magnésium dans un organisme humain ou animal selon l'utilisation définie à la revendication 8, caractérisé en ce que l'agent-vecteur contient de l'acide (DL) - malique ou un de ses sels administrables dans l'organisme, le rapport des concentrations molaires ligand-malate/magnésium étant de préférence de l'ordre de 2.

13. Procédé pour obtenir une composition destinée à l'administration de magnésium dans un organisme humain ou anuimal, caractérisé en ce que l'on associe en quantité efficace deux ingrédients actifs, distincts ou non, de l'acide citrique ou un de ses sels administrables dans l'organisme et un composé apporteur de l'ion Mg2 +, en association avec un excipient ou un véhicule pharmaceutique acceptable.

14. Procédé selon la revendication 13, caractérisé en ce que l'on associe l'ion citrate à titre de ligand d'au moins 25 mg d'ion Mg2 + administrables en solution aqueuse et correspondants à une concentration minimum de 0,005 mol.dm-3, pour former un complexe électriquement neutre de l'ion Mg2 +, selon une concentration minimale de 0,005 mol.dm-3 pour l'ion citrate seul, la concentration citrate du ligand seul étant de préférence égale à 0,16 mol.dm-3 pour une concentration d'ion Mg2 + égale à 0,04 mol.dm-3.

15. Procédé selon la revendication 13, caractérisé en ce que l'on associe de l'acide citrique ou un de ses sels administrables dans l'organisme pour agir en tant que ligand d'au moins 25 mg d'ion Mg2 +, le rapport des concentrations molaires ligand-citrate/magnésium étant de préférence compris entre 1,2 et 4.

16. Procédé selon l'une des revendications 10 à 15, caractérisée en ce que la composition comporte du carbonate de magnésium accompagné éventuellement d'un composé neutralisant en quantité suffisante pour stabiliser entre 4 et 5 le pH de la solution administrable correspondante.

17. Procédé selon l'une des revendications 10 à 16, caractérisée en ce que la composition est conditionnée forme de préparation administrable dans l'organisme sous forme de préparation administrable dans l'organisme par voie orale ou par voie parentérale.

18. Procédé pour obtenir des complexes, notamment complexes électriquement neutres, de l'ion magnésium Mg2 + du type MqLp et/ou du type MqLpHr où M représente l'ion Mg2 +, H représente le proton et L représente un ligand de l'ion Mg2 +, caractérisé en ce que l'on associe en solution aqueuse un composé dissociable apporteur de l'ion Mg2 + avec un ligand choisi dans le groupe suivant: maléate, malonate et malate.

19. Utilisation d'une composition obtenue par mise en oeuvre de l'utilisation selon l'une des revendications 6 à 9 et/ou par le procédé selon l'une des revendications 10 à 17 et/ou d'un complexe ingérable obtenu par le procédé selon la revendication 18 à la magnésothérapie humaine ou vétérinaire et/ou aux supplémentations magnésiennes dans le cadre de régimes diététiques humain et animal.

FIG_1

FIG_2

FIG_3

FIG_4a

FIG_4b

FIG_4c

CMg=0,005 mol.dm-3 CMLO=0,05 mol.dm-3

FIG_4d

CMg=0,005 mol.dm-3 CCa=CMLO=0,05 mol.dm-3

FIG_5

EP 0 401 096 A1

FIG_6

FIG_7

FIG_8

FIG_9

FIG_10

FIG_11

FIG_12

FIG_13

FIG_14

FIG_15

FIG_16

FIG_17

FIG_18

FIG_19

FIG_20

FIG.21

FIG.22

FIG.23

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 364 657 (LEWIS/HOWE CO.) <br> * Page 3, line 9 - page 4, line 9; page 46, lines 20-28; revendications 3-4 * <br> --- | 1-20 | A 61 K 47/12 <br> A 61 K 33/06 |
| A | FR-A-2 520 613 (R.M. MOREAU-LAVAUD) <br> ----- | 1-20 | |

**DOMAINES TECHNIQUES RECHERCHES (Int. Cl.5)**

A 61 K
C 07 C

Le présent rapport a été établi pour toutes les revendications

| Lieu de la recherche | Date d'achèvement de la recherche | Examinateur |
|---|---|---|
| LA HAYE | 14-09-1990 | BRINKMANN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.............................................................................
& : membre de la même famille, document correspondant

EPO FORM 1503 03.82 (P0402)